(19) Europäisches Patentamt
European Patent Office
Office européen des brevets

(11) Veröffentlichungsnummer : **0 042 354**
**B1**

(12) # EUROPÄISCHE PATENTSCHRIFT

(45) Veröffentlichungstag der Patentschrift : **11.12.85**

(21) Anmeldenummer : **81810228.7**

(22) Anmeldetag : **09.06.81**

(51) Int. Cl.⁴ : **C 07 D513/04**, A 61 K 31/55// C07D233/84, C07D403/12 ,(C07D513/04, 285:00, 235:00)

(54) **Imidazobenzothiadiazepine, Verfahren zu ihrer Herstellung sowie pharmazeutische Präparate die diese Verbindungen enthalten.**

(30) Priorität : **12.06.80 US 158671**

(43) Veröffentlichungstag der Anmeldung : **23.12.81 Patentblatt 81/51**

(45) Bekanntmachung des Hinweises auf die Patenterteilung : **11.12.85 Patentblatt 85/50**

(84) Benannte Vertragsstaaten : **AT BE CH DE FR IT LI LU NL SE**

(56) Entgegenhaltungen :
**EP-A- 0 003 016**
**DE-A- 2 349 064**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Vlattas, Isidoros, Dr.**
**131 Butler Parkway**
**Summit New Jersey 07901 (US)**

**EP 0 042 354 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 5-Diazacycloalkyl-imidazo [2,1-b] [1,3,5] benzothiadiazepine der allgemeinen Formel I

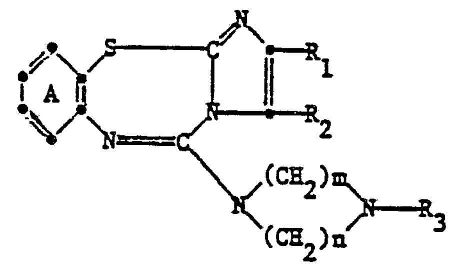

(I)

worin jedes der Symbole $R_1$ und $R_2$ Wasserstoff, $C_{1-7}$-Alkyl, $C_{1-7}$-Alkanoyl, Halogen, Cyan, Carboxy, Carbo-($C_{1-7}$)-alkoxy, Carbamoyl, Sulfamoyl, Mono- oder Di-($C_{1-7}$)-alkyl-(carbamoyl oder sulfamoyl) bedeutet, der Rest A unsubstituiert oder einfach durch $C_{1-7}$-Alkyl, $C_{1-7}$-Alkoxy, $C_{1-7}$-Alkylthio, Halogen, Trifluormethyl, Sulfamoyl, Mono- oder Di-($C_{1-7}$)-alkylsulfamoyl substituiert ist, jedes der Symbole m und n die ganze Zahl 2 oder 3 bedeutet, und $R_3$ für Wasserstoff, $C_{1-7}$-Alkyl, $C_{1-7}$-Alkanoyl, $C_{1-7}$-Alkoxycarbonyl, Phenyl-($C_{1-7}$)-alkoxycarbonyl oder Hydroxy-($C_{1-7}$)-alkyl steht, worin Hydroxy vom Stickstoffatom durch mindestens 2 Kohlenstoffatome getrennt ist, sowie, soweit $R_3$ für $C_{1-7}$-Alkyl oder Hydroxy-($C_{1-7}$)-alkyl steht, entsprechende N-Oxide am N-$R_3$ von Verbindungen der Formel I, und/oder ihre S-Oxide, und die Salze von solchen Verbindungen, insbesondere therapeutisch verwendbare Salze mit Säuren oder Basen, von allen diesen Verbindungen, sowie Verfahren zu ihrer Herstellung, pharmazeutische Präparate enthaltend diese Verbindungen, sowie ihre therapeutische Verwendung.

Eine $C_{1-7}$-Alkylgruppe $R_1$, $R_2$, $R_3$ und/oder eine solche, welche am Ring A sitzt (1,2-Phenylenrest), aber auch in den genannten Alkoxy-, Alkylthio- oder anderen genannten alkylierten Gruppen, ist vor allem Methyl, und auch Aethyl, n- oder iso-(Propyl, Butyl, Pentyl, Hexyl oder Heptyl), z. B. 2-Methylpropyl oder 3-Methylbutyl. $C_{1-7}$-Alkanoyl ist vorzugsweise Acetyl oder Propionyl.

Ein Halogenatom $R_1$ und/oder $R_2$, oder ein solches im Ring A, ist vorzugsweise Fluor oder Chlor, aber auch Brom.

Eine Carbo-($C_{1-7}$)-alkoxy-, Mono- oder Di-($C_{1-7}$)-alkylcarbamoyl- oder Mono- oder Di-($C_{1-7}$)-alkylsulfamoylgruppe $R_1$ und/oder $R_2$, ist vorzugsweise Carbomethoxy, Carbäthoxy ; Mono- oder Dimethylcarbamoyl bzw. Mono- oder Di-methylsulfamoyl. Soweit der Ring A durch Mono- und Dialkylsulfamoylgruppen substituiert ist, ist Mono- oder Dimethylsulfamoyl bevorzugt.

Der Ring A ist vorzugsweise unsubstituiert oder durch die genannten Substituenten monosubstituiert. Solche Substituenten sind z. B. Methyl oder Aethyl ; Methoxy, Aethoxy oder iso-Propoxy ; Methylthio oder Aethylthio ; Fluor, Chlor oder Brom ; Trifluormethyl ; Sulfamoyl, Mono- oder Dimethylsulfamoyl.

Eine Alkylengruppe $(CH_2)_m$ und $(CH_2)_n$ ist insbesondere Aethylen, aber auch 1,2- oder 1,3-Propylen, welche mit den benachbarten Stickstoffatomen vorzugsweise eine Piperazino- oder Homopiperazinogruppe bilden.

Eine Carbo-($C_{1-7}$)-alkoxy- oder Hydroxy-($C_{1-7}$)-alkylgruppe $R_3$ ist vorzugsweise Carbomethoxy oder Carbäthoxy, 2-Hydroxy-(äthyl oder propyl), 3-Hydroxy-(propyl oder butyl) bzw. 4-Hydroxybutyl.

Eine Phenyl-($C_{1-7}$)-alkoxycarbonylgruppe ist z. B. Phenylmethoxycarbonyl oder Phenyläthoxycarbonyl.

Die genannten N-Oxide sind vorzugsweise solche, in welchen $R_3$ $C_{1-7}$-Alkyl oder Hydroxy-($C_{1-7}$)-alkyl bedeutet, und in welchen das Sauerstoffatom an das die Gruppe $R_3$ tragende Stickstoffatom gebunden ist. Die genannten S-Oxide sind Sulfoxide (SO) oder Sulfone ($SO_2$).

Gleichermassen sind die genannten quaternären $C_{1-7}$-Alkylderivate von Verbindungen der Formel I, von solchen abgeleitet, in welchen $R_3 C_{1-7}$-Alkyl oder Hydroxy-($C_{1-7}$)-alkyl bedeutet, und in welchen lediglich das terminale Stickstoffatom des Piperazino- oder Homopiperazino-Ringes quaternisiert ist. Die Anionen der genannten quaternären Derivate, aber auch diejenigen von den genannten Säureadditionssalzen sind vorzugsweise solche, welche therapeutisch verwendbare Salze ergeben, z. B. solche von weiter unten genannten Säuren. Die Verbindungen der Formel I, in welchen $R_1$ und/oder $R_2$ Carboxy bedeutet, bilden auch Salze mit Basen, vorzugsweise solchen, welche therapeutisch verwendbare Salze ergeben, z. B. Ammoniak, Mono-, Di- oder Tri-($C_{1-7}$)-alkylaminen, $C_{1-7}$-Alkylenaminen, Morpholin, Piperazin, Pyridin, oder mit $C_{1-7}$-Alkylderivaten der genannten cyclischen Basen ; Alkalimetall- oder Erdalkalimetallhydroxyden.

Die Verbindungen der Erfindung zeigen wertvolle pharmakologische Eigenschaften, in erster Linie neuroleptische Wirkungen. Diese können in Tierversuchen, vorzugsweise an Säugern, z. B. Mäusen, Ratten oder Affen, als Testobjekte, nachgewiesen werden. Die genannten Verbindungen können ihnen enteral oder parenteral, vorzugsweise oral oder subkutan, intravenös oder intraperitoneal, z. B. durch Steckkapseln oder in Form von Stärke enthaltenden Suspensionen bzw. wässerigen Lösungen,

verabreicht werden. Die verwendete Dosis kann in einem Bereich von 0,01 und 100 mg/kg/Tag, vorzugsweise 0,05 und 10 mg/kg/Tag, insbesondere zwischen 0,1 und 5 mg/kg/Tag, liegen.

Die genannten neuroleptischen Eigenschaften können an ausgewachsenen Ratten oder Totenkopf-Aeffchen (squirrel monkeys) nachgewiesen werden. Die Tiere sind zur Betätigung eines Hebels trainiert, wodurch sie einem auf den Fuss verabreichten elektrischen Schock ausweichen können. Jeder Hebeldruck verschiebt den Schock um 30 Sekunden. Vergisst das Tier den Hebel innerhalb des genannten Zeitintervalls einmal zu drücken, so werden alle 15 Sekunden kurze (0,5 sec.) elektrische Schocks abgegeben, bis dar Tier den Hebel wieder betätigt. Unter Kontrollbedingungen drücken die Versuchstiere den Hebel mit einer mässig ausgeglichenen Geschwindigkeit und erhalten selten mehr als 6 Schocks während einer 25-minuten (Ratten) bis 4-stündigen Versuchsperiode. Die genannten Verbindungen, welche den Tieren 30, 90 und 210 Minuten vor dem Versuch verabreicht werden, blockieren das angelernte konditionierte Vermeidungs-Verhalten. Die Folge ist die Abnahme der Vermeidungs-Reaktion und eine wesentliche Zunahme der von den Tieren erlittenen Schocks. Sowohl die Anzahl der Vermeidungs-Reaktionen als auch diejenige der Fehlverhalten (der erhaltenen Schocks) werden zur Auswertung in diesem Sidman-Vermeidungstest registriert. Die erfindungsgemässen Verbindungen. z. B. das 5-(4-Methylpiperazino)-imidazo [2,1-b] [1,3,5] benzothiadiazepin oder seine therapeutisch verwendbaren Salze, vermindern die Vermeidungs-Reaktionen von Ratten oder Affen in einer Gesamtdosis von 10 mg/kg oder darunter.

Die bei den klassischen Neuroleptica bekannten extrapyramidalen Nebenwirkungen (ENW) lösen in Totenkopf-Aeffchen, welche vorher einer wiederholten antipsychotischen Behandlung unterworfen waren, charakteristische motorische Syndrome aus. Diese Bewegungsstörungen bestehen aus dystonischen Körperhaltungen und dyskinetischen Bewegungen, welche besser den bei Menschen auftretenden ENW entsprechen, als dies die Katalepsie oder Zittern tut. So können potentielle extrapyramidale Nebenwirkungen, aber gegebenenfalls auch andere neurologische Symptome, z. B. Ptosis, durch Beobachtung von ausgewachsenen Totenkopf-Aeffchen (von einem Gewicht von 700-1 200 g) beurteilt werden. Die Tiere werden einmal wöchentlich oder einmal in zwei Wochen mit Haloperidol (1,25 mg/kg) behandelt. Nach einer derartigen Behandlung von ungefähr 2-4 Monaten treten 1-6 Stunden nach der Verabreichung von Haloperidol offensichtliche dystonische Körperhaltungen und dyskinetische Bewegungen auf. Sonst können in anderen Zeitpunkten keine abnormalen Bewegungen der Affen festgestellt werden. Nach dem Auftreten von, durch Haloperidol hervorgerufenen Dyskinesien, wird die oben geschilderte Behandlungsweise abgebrochen. Die Tiere erhalten nachher lediglich alle 4-8 Wochen 1,25 mg/kg Haloperidol und sie werden als Kontrolltiere bei der Untersuchung von Verbindungen dieser Erfindung eingesetzt.

Die Affen werden in Intervallen von 2, 4 und 6 Stunden nach der Behandlung beurteilt. Die Versuche werden in verschlossenen Zellen, welche mit ferngesteuerten Video-Beobachtungsapparaten und mit einem einheitlichen Summton-Generator ausgerüstet sind, durchgeführt. Die Beurteilungen der Tiere werden durch zwei voneinander unabhängige Beobachter vorgenommen, welche keine Kenntnis von den verabreichten Verbindungen und vom Ziel des Versuchs haben. Während der neurologischen Untersuchungen werden die Affen von einer dritten Person behandelt. Zuerst werden die Tiere durch das Video-System beobachtet. Dann betreten die Beobachter die Versuchstelle und notieren die Reaktionen der Affen als « motorisch » (das Tier geht umher in der Zelle), « visuell » (eine offenbare Reaktion, jedoch kein Umhergehen) und « keine » (keine Reaktion). Es werden auch Aktivität, Köperhaltung, Zittern, vermehrter Speichelfluss und andere neurologische Symptome berücksichtigt. Besondere Beachtung wird dem Vorhandensein oder Abwesenheit von « bizarren », vorher genannten, distonischen Körperhaltungen und dyskinetischen Bewegungen gewidmet. Die die Tiere behandelnde Person nimmt dann den Affen aus der Zell heraus. Dabei werden die Reaktionen des Affen auf die Annäherung der mit einem Handschuh geschützten Hand, auf die erste Berührung beim Anfassen und auf die Hemmisse nach der Gefangennahme, separat aufgeschrieben. Die Vokalisation während der Gefangennahme wird auch beurteilt. Die Person, welche das Tier behandelt, wertet dann den Körpertonus und die Pupillengrösse aus und stellt fest, ob Ptosis vorhanden ist. Hat die vorhergehende Untersuchung nahegelegt, dass Katalepsie aufgetreten ist, so wird dieses Symptom ausgewertet. Der Affe wird zuerst, Kopf nach unten, auf den Boden, dann zum Zelleneingang und schliesslich in die Beobachtungszelle gestellt. Bleibt der Affe in allen diesen Stellungen mindestens 5 Sekunden im wesentlichen unbeweglich, so wird das Vorhandensein der Katalepsie bejaht. Nach der Rückkehr des Affen in seine Versuchszelle oder nachdem er dorthin zurückgebracht worden ist, wird er wieder 1 Minute zwecks Feststellung von Dyskinesien beobeachtet. Ein gewisses Symptom oder ein Bewertungsresultat, welches sich von den Kontrollergebnissen unterscheidet, wird erst dann dem verabreichten Mittel zugeschrieben, wenn in einem der drei Beobachtungsperioden die beiden Beobachter darüber berichtet haben. Wenn ein gegebenes Symptom der erste Beobachter für schwächer als der zweite hält, so wird die weniger strenge Einschätzung angenommen. Im allgemeinen ist jedoch die Uebereinstimmung zwischen den Beobachtern gut.

So zeigte zum Beispiel nach Verabreichung von 10 mg/kg der Verbindung des Beispiels 1 lediglich einer von fünf Affen einen gewissen Typus von dyskinetischen Bewegungen. Dieser Fall war lediglich auf eine Form (Krümmung) beschränkt und trat einmal in drei Versuchsperioden auf. Im Gegensatz dazu wird an allen Affen, welche mit Haloperidol (1,25 mg/kg) behandelt worden sind, eine schwere Dyskinesie,

welche sich in verschiedenen Typen von dyskinetischen Bewegungen manifestiert, festgestellt. Dyskinesien sind auch nach Clozapin (10 mg/kg) nicht aufgetreten. Sie sind jedoch bereits nach einer niedrigen Dosis von Haloperidol (0,625 mg/kg) an fünf Affen feststellbar. Nach Verabreichung vom pharmazeutischen Vehikel (Trägermaterial) sind nie Dyskinesien aufgetreten. Clozapin hat jedoch in diesem Test bei jedem untersuchten Affen einen vermehrten Speichelfluss hervorgerufen. Diese Nebenwirkung war nicht festellbar bei der Behandlung mit anderen Verbindungen, inklusive das Produkt des Beispiels 1. Ptosis, Katalepsie, Verminderung vom Körpertonus und schwächere Reaktionen gegenüber den Beobachtern hatte man in einigen oder allen Affen nach der Verabreichung der genannten drei Wirkstoffe festgestellt.

Gemäss den genannten, und auch anderen klassischen Testmethoden, sind die Verbindungen der Erfindung wertvolle neuroleptische (antipsychotische) Mittel, welche zum Beispiel in der Behandlung oder Handhabung von Aggressionen, Aufregungs- oder Angstzuständen verwendet werden können. Sie sind praktisch frei von extrapyramidalen Nebenwirkungen, welche z. B. Clozapin in Menschen zeigt. Ueberdies bedeutet die Abwesenheit von vermehrtem Speichelfluss einen weiteren Vorteil der neuen Verbindungen gegenüber Clozapin. Die erfindungsgemässen Verbindungen können überdies als Zwischenprodukte zur Herstellung von anderen wertvollen, insbesondere von pharmakologisch wirksamen Präparaten, eingesetzt werden.

Bevorzugt sind Verbindungen der allgemeinen Formel II

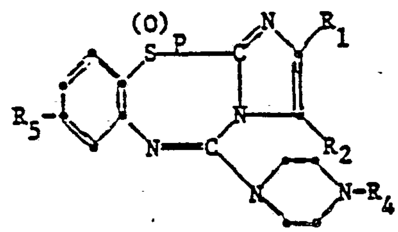

(II)

worin jedes der Symbole $R_1$ und $R_2$ Wasserstoff oder $C_{1-7}$-Alkyl bedeutet, $R_4$ für Wasserstoff, $C_{1-7}$-Alkyl oder 2- oder 3-Hydroxy-($C_{1-7}$)-alkyl steht, $R_5$ Wasserstoff, $C_{1-7}$-Alkyl, $C_{1-7}$-Alkoxy, Halogen oder Trifluormethyl bedeutet, und p für die Zahl von 0 bis 2 steht, ihre N-Oxide, und Salze, insbesondere therapeutisch verwendbare Salze, von allen diesen Verbindungen.

Besonders hervorzuheben sind Verbindungen der allgemeinen Formel II, worin jedes der Symbole $R_1$ und $R_2$ Wasserstoff oder Methyl bedeutet, $R_4$ für $C_1$-$C_4$ Alkyl oder 2- oder 3-Hydroxyalkyl, steht, $R_5$ Wasserstoff, Methyl, Methoxy, Fluor, Chlor oder Trifluormethyl bedeutet, und p für Null sthet, ihre N-Oxide, und Salze, insbesondere therapeutisch verwendbare Salze, von allen diesen Verbindungen.

Die Verbindungen der Erfindung werden nach an sich bekannten Methoden, z. B. dadurch hergestellt, dass man

a) Verbindungen der allgemeinen Formel III oder ihre Salze mit Verbindungen der allgemeinen Formel IV kondensiert

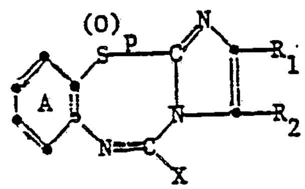

(III)

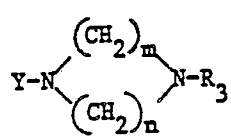

(IV)

worin X Halogen, Niederalkoxy, Niederalkylthio, Cyanato oder Thiocyanato bedeutet, Y für Wasserstoff oder ein Alkalimetallatom steht, p eine Zahl von Null bis 2 bedeutet, und die anderen Symbole die für die Formel I angegebenen Bedeutungen haben, und, wenn erwünscht, eine erhaltene Verbindung in eine andere Verbindung der Formel I umwandelt.

Die genannte Kondensation wird vorzugsweise mit einem Ueberschuss des Piperazins IV (Y=H), oder mit äquivalenten Mengen seiner genannten Metallderivate, vorzugsweise wenn X Halogen, Niederalkylthio oder Thiocyanato bedeutet, durchgeführt. Man arbeitet vorzugsweise bei Temperaturen zwischen ungefähr 0° und 150 °C und in einem geeigneten Lösungsmittel, wie in einem Niederalkanol, z. B. Amylalkohol, oder Dimethylformamid, Hexamethylphosphoramid oder Toluol.

Ein weiteres Verfahren zur Herstellung von Verbindungen der allgemeinen Formel I besteht darin, dass man

b) Verbindungen der allgemeinen Formel V

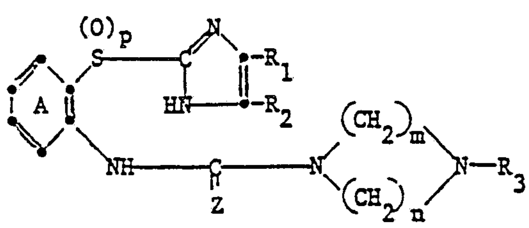

(V)

worin Z ein Sauerstoff- oder Schwefelatom oder NH bedeutet, und die anderen Symbole die oben angegebenen Bedeutungen haben, unter entwässernden oder dehydrosulfurierenden Bedingungen ringschliesst, und, wenn erwünscht, eine erhaltene Verbindung in eine andere Verbindung der Erfindung umwandelt.

Der genannte Ringschluss wird mit starken Dehydratisierungs- oder Dehydrosulfurierungsmitteln, z. B. mit Phosphorhalogeniden und/oder -oxyhalogeniden oder Cyanhalogeniden, mit oder ohne Kronenäther-Katalysatoren, z. B. 8-Kronen-6-äther, und in Gegenwart oder Abwesenheit von basischen Katalysatoren, wie Triäthylamin oder Kaliumcarbonat, vorzugsweise in einem inerten Lösungsmittel, z. B. Dimethylformamid, durchgeführt.

Der Ausdruck « nieder » definiert in den oben oder nachfolgend genannten organischen Resten oder Verbindungen solche mit höchstens 7 Kohlenstoffatomen, vorzugsweise 4, insbesondere 1 oder 2 Kohlenstoffatomen.

Die Ausgansstoffe sind bekannt oder können, falls sie neu sind, nach an sich bekannten Methoden erhalten werden. z. B. analog wie in den Beispielen beschrieben.

Die Imidazo [2,1-b] [1,3,5] benzothiadiazepin-Ausgangsstoffe der Formel III werden nach an sich bekannten Ringschluss-Methoden hergesellt. Vorzugsweise kondensiert man Verbindungen der Formel VI

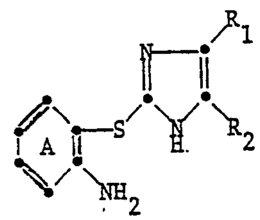
(VI)

worin A, $R_1$ und $R_2$ die für die Verbindungen der Formel III angegebenen Bedeutungen haben, mit reaktionsfähigen Kohlensäure-Derivaten, wie Phosgen, Thiophosgen, 1,1'-Carbonyldiimidazol oder cyan.

Verbindungen der Formel III, worin X Hydroxy bedeutet, können in solche, in welchen X für Sulfhydryl steht, mit konventionellen Sulfurierungsmitteln, z. B. mit Phosphorpentasulfid umgewandelt werden. Diese können in Verbindungen, in welche X die obige Bedeutung hat, analog zu den in den Beispielen beschriebenen Methoden übergeführt werden.

Die Ausgangsstoffe der Formel V können ausgehend von den (tautomeren) Vorstufen von Verbindungen der Formel III, worin X Hydroxy, Thio oder Amino bedeutet, hergestellt werden. Diese Verbindungen werden mit Verbindungen der Formel IV in Gegenwart oder Abwesenheit von anderen Basen, z. B. den oben genannten, vorzugsweise in inerten Lösungsmitteln, wie Methylenchlorid oder Toluol, bei Temperaturen zwischen 0° und 150 °C, vorzugsweise zwischen 10° und 50 °C, kondensiert. Das Ringöffnen wird vorzugsweise bei niedrigen Temperaturen, um Nebenreaktionen der gegebenenfalls vorhandenen reaktionsfähigen funktionellen Gruppen $R_1$ und $R_2$ vorzubeugen, durchgeführt.

Auf einem anderen Wege können Ausgangsstoffe der Formel V, in welchen $R_3$ $C_{1-7}$-Alkanoyl, $C_{1-7}$-Alkoxycarbonyl oder Phenyl-($C_{1-7}$)-alkoxycarbonyl bedeutet, durch Kondensation von Verbindungen der Formel VI mit einer Verbindung der Formel VII

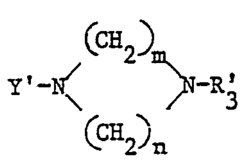
(VII)

worin Y' Halogencarbonyl, Halogenthiocarbonyl oder Cyan bedeutet, vorzugsweise in einem inerten Lösungsmittel, bei Temperaturen zwischen 0° und 150 °C, hergestellt werden.

Die erhaltenen Verbindungen der Erfindung können in an sich bekannter Weise in andere Verbindungen der Erfindung übergeführt werden. So können z. B. Verbindungen, in welchen $R_3$ Wasserstoff oder ein Alkalimetallatom bedeutet, z. B. ihre Natrium- oder Lithiumsalze, mit substituierten oder unsubstituierten Oxiranen, z. B. mit Aethylenoxyd, oder mit reaktionsfähigen Estern von unsubstituierten oder entsprechend substituierten aliphatischen oder araliphatischen Alkoholen, z. B. Methanol, Aethanol, Allyl- oder Propargylalkohol, in die entsprechenden N-substituierten Verbindungen oder quaternären Derivate, je nach der molaren Menge des verwendeten Alkylierungsmittel, übergeführt werden. Diese Ester sind z. B. von starken anorganischen oder organischen Säuren, vor allem von Halogenwasserstoffsäuren, z. B. Chlorwasserstoff-, Bromwasserstoff- oder Jodwasserstoffsäure, Schwefelsäure oder einer aromatischen Sulfonsäure, z. B. p-Toluolsulfonsäure oder m-Brombenzolsulfonsäure abgeleitet. Zwischenprodukte der Formel I, in welchen $R_3$ ein Alkalimetallatom bedeutet, können durch

Metallisierung mit reaktionsfähigen organischen Metallverbindungen, z. B. Lithium-diisopropylamid, mit Alkalimetall-alkoxiden, z. B. Natrium-methoxid, oder mit Alkalimetallhydriden, z. B. Natrium- oder Kaliumhydrid, erhalten werden.

Verbindungen der Formel I, in welchen $R_3$ Hydroxy-($C_{1-7}$)-alkyl bedeutet, können auch dadurch hergestellt werden, dass man entsprechende Verbindungen der Formel I, in welchen $R_3$ Wasserstoff bedeutet, zuerst mit reaktionsfähigen Derivaten von entsprechenden Glykolen, Glykolsäuren oder Dicarbonsäuren, z. B. ihren Niederalkylestern, Halogeniden der Anhydriden, oder mit reaktionsfähigen Estern der genannten Glykole oder Glykolsäurederivate, z. B. mit Estern von Halogenwasserstoffsäuren oder aromatischen Sulfonsäuren, 1,2-Dibromäthan oder -1,2-Dibrompropan, Bromessigsäure-äthylester oder Brompropionsäure-äthylester, Tosyloxyessigsäure-äthylester, Oxalsäurediäthylester, Malonsäurediäthylester oder Oxalsäuremonoäthylester-chlorid umsetzt. Die erhaltenen Zwischenprodukte werden entweder hydrolysiert oder mit einfachen oder komplexen Leichtmetallhydriden, z. B. Lithiumaluminiumhydrid, allein oder mit Diboran, zu den Verbindungen der Formel I, in welchen $R_3$ Hydroxy-($C_{1-7}$)-alkyl bedeutet, reduziert.

Verbindungen der Formel I, in welchen $R_3$ Methyl bedeutet, können wie folgt hergestellt werden : Zuerst werden Verbindungen, in welchen $R_3$ für Wasserstoff steht, durch Umsetzung mit Halogenameisensäureniederalkylestern oder -phenylniederalkylester, z. B. Chlorameisensäure-äthylester, in die Verbindungen der Formel I, worin $R_3$ $C_{1-7}$-Alkoxycarbonyl oder Phenyl-($C_{1-7}$)-alkoxycarbonyl bedeutet, übergeführt Dann reduziert man diese Acylderivate mit einfachen oder komplexen Leichtmetallhydriden, z. B. mit Lithiumaluminiumhydrid, Natrium-tri-tert.-butoxy-aluminiumhydrid oder Natrium-bis-(2-methoxy-äthoxy)-aluminiumhydrid.

N-Acylderivate können aus Verbindungen der Formel I, in welchen $R_3$ Wasserstoff bedeutet und entsprechenden reaktionsfähigen Säurederivaten, z. B. Säurehalogeniden, einfachen oder aktivierten Estern, z. B. Alkylestern oder Cyanalyklestern, Anhydriden oder Isocyanaten, erhalten werden. Erhaltene Verbindungen der Formel I, in welchen $R_1$ und/oder $R_2$ Wasserstoff bedeutet, können in die entsprechenden 3- und/oder 4-(Halogen oder Acyl)-Derivate, z. B. durch Halogenierung, vorzugsweise mit Chlor in Essigsäure oder unter Bedingungen der Friedel-Crafts-Reaktion, und/oder durch Acylierung mit einem Trihalogenacetyl-halogenid oder einer Halogensulfonsäure, und nachfolgende Behandlung mit einem Aklalimetall-niederalkoxyd, -hydroxyd oder -amid, umgewandelt werden. Erhaltene Carbonsäure- oder Sulfonsäure-Derivate können dann in bekannter Weise, vorzugsweise unter alkalischen Bedingungen hydrolysiert und/oder mit Ammoniak, Mono- oder Di-niederalkylaminen amidiert werden. Die erhaltenen Carboxamide können nach konventionellen Methoden zu den entsprechenden Nitrilen dehydratisiert werden.

Erhaltene tertiäre Stickstoff-Verbindungen, in welchen $R_3$ sich vom Wasserstoff unterscheidet, können in N- und/oder S-Oxide umgewandelt werden. Man arbeitet z. B. mit Wasserstoffsuperoxyd oder organischen Persäuren, z. B. niederen Peralkansäuren oder Perbenzoesäuren, wie Peressigsäure oder m-Chlor-perbenzoesäure, vorzugsweise bei Zimmertemperatur oder mit der letztgenannten Säure, darunter oder bis 100 °C mit verdünntem Wasserstoffsuperoxyd, in Gegenwart von Niederalkansäuren, z. B. Essigsäure. Sind lediglich N-Oxide erwünscht, so ist, insbesondere mit den genannten Persäuren, vorsichtig vorzugehen, um der S-Oxidation bei zu langer Reaktionsdauer vorzubeugen.

Sind lediglich S-Oxide erwünscht, so werden Verbindungen, in welchen $R_3$ Acyl, z. B. $C_{1-7}$-Alkoxycarbonyl oder Phenyl-($C_{1-7}$)-alkoxycarbonyl bedeutet, mit Wasserstoffsuperoxyd oder organischen Persäuren, vorzugsweise mit m-Chlor-perbenzoesäure, behandelt.

Man arbeitet vorzugsweise bei Zimmertemperatur oder darunter, um je nach der Menge der verwendeten Persäure, Sulfoxide (SO) oder Sulfone ($SO_2$) zu erhalten. Die so erhaltenen Verbindungen, in welchen $R_3$ Phenyl-($C_{1-7}$)-alkoxycarbonyl oder $C_{1-7}$-Alkoxycarbonyl bedeutet, können gemäss den an sich bekannten, vorher beschriebenen Methoden, in andere Verbindungen der Formel I übergeführt werden.

Schliesslich können die Verbindungen der Erfindung in Form von freien Basen oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z. B. durch Behandlung mit einer stärkeren Base, wie mit einem Metallhydroxyd oder Ammoniumhydroxyd, basischen Salz oder einem Kationenaustauscher, z. B. mit einem Alkalimetallhydroxyd- oder -carbonat, umgewandelt werden. Säuren, die therapeutisch verwendbare Säureadditionssalze ergeben, sind z. B. anorganische Säuren, wie Halogenwasserstoffsäuren, z. B. Chlorwasserstoff- oder Bromwasserstoffsäure, oder Schwefel-, Phosphor-, Salpeter- oder Perchlorsäure ; oder vorzugsweise organische Säuren, wie aliphatische oder aromatische Carbon- oder Sulfonsäuren, z. B. Ameisen-, Essig-, Propion-, Bernstein-, Glykol-, Milch-, Aepfel-, Wein-, Glucon-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Aethylensulfon-, Benzolsulfon-, Toluolsulfon-, Naphthalinsulfon-, Sulfanil- oder Cyclohexylsulfaminsäure ; oder die Ascorbinsäure. Diese oder andere Salze, z. B. die Pikrate, können auch in der Reinigung von freien Basen verwendet werden. Die Basen werden in ihre Salze übergeführt, die Salze abgetrennt und die Basen aus den Salzen freigesetzt.

Infolge der engen Beziehung zwischen den neuen Verbindungen in Freier Form und in Form ihrer

Salze sind im vorausgegangenen und nachfolgend unter freien Verbindungen und Salzen sinn- und zweckgemäss gegebenenfalls auch die entsprechenden Salze bzw. freien Verbindungen zu verstehen.

Erhaltene Isomerengemische von Verbindungen, z. B. solchen der Formel I bis VII, können nach an sich bekannten Methoden, z. B. durch fraktionierte Destillation, Kristallisation und/oder Chromatographie, in die einzelnen Isomeren getrennt werden. Razemische Produkte können in die optischen Antipoden, z. B. bei Trennung ihrer diastereomeren Salze, z. B. durch fraktionierte Kristallisation der d- oder l-Tartrate getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stude abgebrochen wird, oder wonach ein Ausgangsmaterial unter den Reaktionsbedingungen gebildet, oder worin ein Ausgangsstoff in Form eines Salzes oder reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel II, führen.

Die Verbindungen der Formel I bzw. II können zur Herstellung von pharmazeutischen Präparaten verwendet, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthylenglykol, aufweisen ; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer erhalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von 0,1 % bis 75 %, insbesondere von 1 % bis 50 % des Aktivstoffes. Enzeldosen für Säuger mit einem Gewicht von ungefähr 50-70 kg können zwischen ungefähr 5-100 mg des aktiven Bestandteils enthalten.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln vermindertem Druck, z. B. zwischen 15 und 100 mm/Hg, durchgeführt.

## Beispiel 1

Ein Gemisch von 501 g 1-Methylpiperazin und 6 000 ml Amylalkohol wird mit 538 ml 9,3-normalem methanolischem Chlorwasserstoff versetzt und das Gemisch unter Rühren 1 Stunde destilliert. Während dieser Zeit fängt man 1 000 ml Destillat auf und die Temperatur erreicht 131°. Dann gibt man weitere 501 g 1-Methylpiperazin und 618,5 g 5-Methylthio-imidazol [2,1-b] [1,3,5] benzothiadiazepin dazu. Das Gemisch wird unter Stickstoff bei 132-140° 48 Stunden gerührt und bei ungefähr 80-90° eingedampft. Der Rückstand wird in 3 000 ml Methylenchlorid gelöst, die Lösung 3-mal mit 1 000 ml 3-normaler wässeriger Natriumhydroxydlösung und 5-mal mit 1 000 ml Wasser gewaschen. Schliesslich extrahiert man 4-mal mit je 750 ml 2-normaler Chlorwasserstoffsäure. Die vereinigten Extrakte werden einmal mit 1 000 ml Methylenchlorid gewaschen, mit 75 g Aktivkohle entfärbt, filtriert und das Filtrat wird mit 500 ml 29,9 %-igem wässerigem Ammoniak auf einen pH-Wert von 9-10 eingestellt. Das Gemisch wird zweimal mit 2 000 ml Methylenchlorid extrahiert, die vereinigten Extrakte werden getrocknet, filtriert und bei ungefähr 60° eingedampft. Man löst 2 810 g des Rückstands in 14 000 ml heissem Isopropanol, behandelt die Lösung mit 563 g Aktivkohle, filtriert sie und wäscht den Rückstand mit 1 000 ml kaltem Isopropanol. Die vereinigten Filtrate werden wieder erwärmt und mit 563 g Aktivkohle in gleicher Weise behandelt. Die erhaltene klare Lösung wird auf 8 000 ml konzentriert und das Konzentrat im Kühlschrank 2 Tage stehen gelassen. Der weisse Niederschlag wird abfiltriert, 3-mal mit kaltem Isopropanol gewaschen und bei 40°/5 mmHg getrocknet. Man erhält das 5-(4-Methylpiperazino)-imidazo [2,1-b] [1,3,5] benzothiadiazepin der Formel

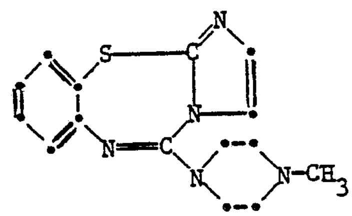

welches bei 145-147° schmilzt.

Man löst 1 614 g dieses Produktes bei 50-60° in 5 450 ml wasserfreiem Aethanol und filtriert die heisse Lösung. Das Filter wird mit weiteren 1 000 ml wasserfreiem Aethanol nachgewaschen und die vereinigten Filtrate werden unter Rühren mit 688 g Maleinsäure in 1 600 ml wasserfreiem Aethanol angesäuert. Das Gemisch wird unter Kühlen auf 25° gerührt, der Niederschlag abgetrennt, zweimal mit 800 ml wasserfreiem Aethanol gewaschen und bei 75°/0,5 mmHg getrocknet. Man erhält das entsprechende Monomaleat, welches unter Zersetzung bei 198-199° schmilzt.

Der Ausgangsstoff wird wie folgt hergestellt : Man gibt unter Rühren in einer Stickstoffatmosphäre innerhalb 45 Minuten 7 500 g Aminoacetaldehyd-dimethylacetal zu 39 230 ml 2-normaler Chlorwasserstoffsäure und dann auf einmal 6 932 g Kaliumthiocyanat. Das Gemisch wird auf 98° erhitzt und 2 Stunden gerührt. Man lässt es über Nacht auf Zimmertemperatur abkühlen. Die erhaltene Suspension wird gerührt und auf 5° gekühlt, filtriert und der Rückstand bei 60°/5 mmHg getrocknet. Man erhält das Imidazol-2-thiol, welches bei 224-226° schmilzt.

Man gibt zuerst 2 468 g der letztgenannten Verbindung zu einer Lösung von 1 604 g 86,9 %-iger wässeriger Kaliumhydroxydlösung in 24 700 ml Isopropanol unter Rühren in einer Stickstoffatmosphäre bei Zimmertemperatur und dann 4 986 g 2-Brom-nitrobenzol. Das Gemisch wird bei 82-85° 5 Stunden gerührt, auf 50° gekühlt und mit 37 000 ml Wasser verdünnt. Die erhaltene Suspension wird bei Zimmertemperatur 2 Tage gerührt, filtriert, der Rückstand 6-mal mit 4 000 ml Wasser und 5-mal mit 3 700 ml Diäthyläther gewaschen und bei 60°/5 mmHg getrocknet. Man erhält das 2-(o-Nitrophenylthio)-imidazol, welches bei 178-180° schmilzt.

Ein Gemisch von 2 210 g der letztgenannten Verbindung, 2 000 ml Wasser, 2 000 ml Aethanol und 1 700 g Einsenpulver wird unter Rühren in einer Stickstoffatmosphäre auf 70° erhitzt. Nach der Hinzufügung von 10 ml konz. Chlorwasserstoffsäure wird das Gemisch 1,5 Stunden unter Rückfluss gekocht und dann innerhalb 95 Minuten mit 200 ml konz. Chlorwasserstoffsäure in 1 000 ml Aethanol versetzt. Das Gemisch wird weitere 2 Stunden unter Rückfluss gekocht und mit 400 ml 6-normaler wässeriger Natriumhydroxydlösung versetzt. Die erhaltene Suspension wird mit 2 000 ml Methanol verdünnt, filtriert und der Rückstand 3-mal mit 1 000 ml Methanol gewaschen. Die vereinigten Filtrate werden mit 40 000 ml Wasser verdünnt und die erhaltene Suspension lässt man sich über Nacht absetzen. Der Niederschlag wird abgetrennt, zweimal mit 2 000 ml Wasser gewaschen und bei 60°/5 mmHg getrocknet. Man erhält das 2-(o-Aminophenylthio)-imidazol, welches bei 137-138° schmilzt.

Ein Gemisch von 55 000 ml Methylenchlorid und 6 975 ml Triäthylamin wird mit 4 775 g 2-(o-Aminophenylthio)-imidazol versetzt, auf 5° gekühlt und innerhalb 2,5 Stunden, unter Rühren in einer Stickstoffatmosphäre bei 15°, mit 3 301 g 85 %-igem Thiophosgen in Tetrachlorkohlenstoff versetzt. Das Gemisch wird bei 10° 4 Stunden und über Nacht bei Zimmertemperatur weitergerührt. Die erhaltene Suspension wird filtriert, der Rückstand zweimal mit 4 000 ml Methylenchlorid und einmal mit 20 000 ml Wasser gewaschen und in 11 000 ml 1,3-normaler Chlorwasserstoffsäure suspendiert. Die Suspension wird 2 Stunden gerührt, filtriert, der Rückstand 3-mal mit 4 000 ml Wasser gewaschen und bei 60°/5 mmHg getrocknet. Man erhält das Imidazo [2,1-b] [1,3,5]-benzothiadiazepin-5(6H)-thion, welches bei 156-159° schmilzt.

Man gibt 1 184 g der letztgenannten Verbindung zu einer Lösung von 278 g Natrium-methoxid in 22 500 ml Isopropanol und rührt das Gemisch 1,5 Stunden in einer Stickstoffatmosphäre. Das Gemisch wird dann innerhalb 30 Minuten mit 791 g Methyljodid versetzt und 3,5 Stunden bei ungefähr 20° weiter gerührt. Das Reaktionsgemisch wird mit 45 000 ml Wasser verdünnt und die erhaltene Suspension über Nacht bei Zimmertemperatur gerührt. Nach Filtration wird der Rückstand 5-mal mit 4 000 ml Wasser gewaschen und bei 60°/5 mmHg getrocknet. Man erhält das 5-Methylthio-imidazo [2,1-b] [1,3,5] benzothiadiazepin, welches bei 116-118° schmilzt.

Ausgehend von entsprechend substituierten 2-Brom-nitrobenzolen werden in analoger Weise die folgenden Ausgangsstoffe hergestellt :

a) 8-Methoxy-5-methylthio-imidazo [2,1-b] [1,3,5] benzothiadiazepin, F. 143-146° ;
b) 8-Chlor-5-methylthio-imidazo [2,1-b] [1,3,5] benzothiadiazepin, F. 147-149° ;
c) 8-Fluor-5-methylthio-imidazo [2,1-b] [1,3,5] benzothiadiazepin, F. 174-176° ;
d) 8-Methyl-5-methylthio-imidazo [2,1-b] [1,3,5] benzothiadiazepin.

Beispiel 2

Eine Lösung von 480 mg 5-Thiocyanato-imidazo [2,1-b] [1,3,5]-benzothiadiazepin in 1 ml Hexamethylphosphoramid wird in einer Stickstoffatmosphäre unter Rühren bei − 5° mit 500 mg 1-Methylpiperazin

innerhalb 5 Minuten versetzt. Das Rühren wird 5 Minuten bei der genannten Temperatur und 15 Minuten bei Zimmertemperatur fortgesetzt. Das Gemisch wird mit 80 ml Essigsäureäthylester verdünnt, zweimal mit gesättigter wässeriger Natriumchloridlösung gewaschen, getrocknet und eingedampft. Der Rückstand wird in 2 ml Aceton aufgelöst, die Lösung mit 300 mg Maleinsäure angesäuert und mit Diäthyläther verdünnt. Man erhält das 5-(4-Methylpiperazino)-imidazo [2,1-b] [1,3,5] benzothiadiazepin-monomaleat, welches unter Zersetzung bei 198-199° schmilzt. Das Produkt ist mit demjenigen des Beispiels 1 identisch.

Der Ausgangsstoff wird wie folgt hergestellt : Eine Suspension von 1,44 g 50 %-igem Natriumyhydrid in Mineralöl und 150 ml trockenem Tetrahydrofuran wird portionenweise mit 6,45 g Imidazo [2,1-b] [1,3,5] benzothiadiazepin-5(6H)-thion versetzt und das Gemisch in einer Stickstoffatmosphäre bei Zimmertemperatur eine Stunde gerührt. Die erhaltene weisse Suspension wird auf 0° gekühlt und die Lösung tropfenweise mit 3,5 g Bromcyan in 10 ml Tetrahydrofuran versetzt. Das Gemisch wird bei Zimmertemperatur 0,5 Stunden gerührt und eingedampft. Der Rückstand wird mit Methylenchlorid trituriert, das Gemisch mit Wasser gewaschen, getrocknet, zu einem kleinen Volumen konzentriert, mit Diäthyläther gewaschen und filtriert. Man erhält das 5-Thicyanato-imidazo [2,1-b] [1,3,5] benzothiadiazepin, welches bei 111-113° schmilzt.

Beispiel 3

Ein Gemisch von 333 mg 1-[2-(Imidazo-2-ylthio)-phenyliminothiocarbonyl]-4-methylpiperazin, 3,3 ml Dimethylformamid, 276 mg Kaliumcarbonat, 116 mg Bromcyan und 50 mg 8-Kronen-6-äther wird unter Stickstoff bei Zimmertemperatur 3 Stunden gerührt. Das Gemisch wird mit Essigsäureäthylester verdünnt, mit gesättigter wässeriger Natriumchloridlösung gewaschen, getrocknet und eingedampft. Der Rückstand wird in Aceton gelöst, die Lösung mit 116 mg Maleinsäure behandelt und mit Diäthyläther verdünnt. Man erhält das 5-(4-Methylpiperazino)-imidazo [2,1-b] [1,3,5] benzothiadiazepin-monomaleat, welches unter Zersetzung bei 198-199° schmilzt. Das Produkt ist mit demjenigen des Beispiels 1 identisch.

Der Ausgangsstoff wird wie folgt hergestellt : Ein Gemisch von 2,3 g Imidazo [2,1-b] [1,3,5] benzothiadiazepin-5(6H)-thion, 23 ml Methylenchlorid und 1,0 g 1-Methylpiperazin wird bei Zimmertemperatur 15 Stunden gerührt. Das erhaltene kristalline Produkt wird abfiltriert und mit Methylchlorid gewaschen. Man erhält das 1-[2-(Imidazo-2-ylthio)-phenyliminothiocarbonyl]-4-methylpiperazin, welches bei 209-212° schmilzt.

Beispiel 4

Eine Suspension von 3,1 g 1-[2-(Imidazo-2-ylthio)-phenyliminocarbonyl]-4-methylpiperazin und 25 ml Phosphoroxychlorid wird mit 2,04 g phosphorpentachlorid auf einmal versetzt und das Gemisch bei Zimmertemperatur 4 Stunden gerührt. Es wird eingedampft, der Rückstant in 50 ml trockenem Methylenchlorid suspendiert, die Suspension auf 0° gekühlt und unter Rühren mit 2,02 g Triäthylamin tropfenweise versetzt. Es wird 15 Minuten bei 0° weiter gerührt, das Gemisch mit Wasser gewaschen, getrocknet und eingedampft. Der Rückstand wird in Aceton gelöst und die Lösung mit Maleinsäure angesäuert. Man erhält das 5-(4-Methylpiperazino)-imidazo [2,1-b] [1,3,5] benzothiadiazepin-monomaleat, welches unter Zersetzung bei 198-199° schmilzt. Das Produkt ist mit demjenigen des Beispiels 1 identisch.

Der Ausgangsstoff wird wie folgt hergestellt : Ein Gemisch von 15 g 2-(Imidazo-2-ylthio)-anilin, 13,9 g 1,1'-Carbonyldiimidazol und 675 ml Methylenchlorid wird 24 Stunden bei Zimmertemperatur gerührt. Das erhaltene feste Material wird abfiltriert und mit Methylenchlorid gewaschen. Man erhält das Imidazo [2,1-b] [1,3,5] benzothiadiazepin-5(6H)-on, welches unter Zersetzung bei 250-252° schmilzt.

In analoger Weise (oder nach Austausch von 1,1'-Carbonyldiimidazol durch die äquivalente Phosgenmenge) erhält man die folgenden Zwischenprodukte :

a) 3,4-Dimethylimidazo [2,1-b] [1,3,5] benzothiadiazepin-5(6H)-on, F. 225° (unter Zersetzung) ;
b) 8-Chlorimidazo [2,1-b] [1,3,5] benzothiadiazepin-5(6H)-on, F. 261-263° ;
c) 8-Trifluoromethylimidazo [2,1-b] [1,3,5] benzothiadiazepin-5(6H)-on, F. 257-260° ;
d) 3-Methylimidazo [2,1-b] [1,3,5] benzothiadiazepin-5(6H)-on, F. 225-229°.

Ein Gemisch von 2,17 g Imidazo [2,1-b] [1,3,5] benzothiadiazepin-5(6H)-on, 1,0 g 1-Methylpiperazin und 20 ml Methylenchlorid wird 24 Stunden bei Zimmertemperatur gerührt. Das erhaltene kristalline Material wird abfiltriert und mit Methylenchlorid gewaschen. Man erhält das 1-[2-(Imidazo-2-ylthio)-phenyliminocarbonyl]-4-methylpiperazin, welches bei 197-200° schmilzt.

In analoger Weise werden auch die folgenden, das Verfahren illustrierenden Ausgangsstoffe erhalten :

a) 1-[2-(4-Methylimidazo-2-ylthio)-phenyliminocarbonyl]-4-methylpiperazin, F. 101-105° (Zersetzung) ;
b) 1-[2-(Imidazo-2-ylthio)-phenyliminocarbonyl]-4-methylhomopiperazin, F. 134-138° ;
c) 1-[2-(Imidazo-2-ylthio)-phenyliminocarbonyl]-4-carboäthoxypiperazin, F. 161-170° ;
d) 1-[2-(Imidazo-2-ylthio)-phenyliminocarbonyl]-4-carbobenzoxypiperazin, F. 206-208° ;

e) 1-[2-(4-Carboäthoxyimidazo-2-ylthio)-phenyliminocarbonyl]-4-methylpiperazin, F. 166-169° ;
f) 1-[2-(Imidazo-2-ylthio)-4-trifluoromethyl-phenyliminocarbonyl]-4-methylpiperazin, F. 212-214°.

Beispiel 5

Ein Gemisch von 10 g 5-Methylthio-imidazo [2,1-b] [1,3,5]-benzothiadiazepin-hydrochlorid, 3,62 g Piperazin und 350 ml Amylalkohol wird unter Rühren 20 Stunden in einer Stickstoffatmosphäre unter Rückfluss gekocht. Das Lösungsmittel wird unter vermindertem Druck eingedampft, der Rückstand mit Methylenchlorid trituriert, mit 2-normaler wässeriger Natriumhydroxydlösung gewaschen, über Magnesiumsulfat getrocknet und zur Trockene eingedampft. Der Rückstand wird in 10 ml Methanol gelöst und mit 2-normaler wässeriger Chlorwasserstoffsäure behandelt. Man erhält das 5-(4H-Piperazino)-imidazo [2,1-b] [1,3,5]-benzothiadiazepin-dihydrochlorid, welches bei 249° unter Zersetzung schmilzt.

Beispiel 6

Ein Gemisch von 5 g 5-Methylthio-imidazo [2,1-b] [1,3,5]-benzothiadiazepin-hydrochlorid, 2,86 g N-β-Hydroxyäthylpiperazin und 175 ml Amylalkohol wird 48 Stunden in einer Stickstoffatmosphäre, unter Rühren unter Rückfluss gekocht. Das Lösungsmittel wird unter vermindertem Druck eingedampft, der Rückstand mit Methylenchlorid trituriert, mit 2-normaler wässeriger Natriumhydroxydlösung gewaschen, über Magnesiumsulfat getrocknet und zur Trockene eingedampft. Der Rückstand wird in 5 ml Methanol gelöst und mit 2-normaler ätherischer Chlorwasserstoffsäure behandelt. Man erhält das 5-(4-β-Hydroxyäthylpiperazino)-imidazo [2,1-b] [1,3,5]-benzothiadiazepin-dihydrochlorid, welches bei 210-212° schmilzt.

Beispiel 7

Durch Ersetzung des N-β-Hydroxyäthylpiperazins im Beispiel 6 durch eine äquivalente Menge N-Methylhomopiperazin erhält man das 5-(4-Methylhomopiperazino)-imidazo [2,1-b] [1,3,5] benzothiadiazepin, welches als Fumarat isoliert wird. F. 216-218°.

Beispiel 8

Gemäss den in den vorhergehenden Beispielen illustrierten Methoden werden auch die folgenden Verbindungen der Formel I, ausgehend von äquivalenten Mengen entsprechender Ausgangsstoffe, erhalten ; Ring A = 4-$R_5$-1,2-phenylen ; $(CH_2)_m = (CH_2)_2$ ; $(CH_2)_n = (CH_2)_{n'}$.

| Nr. | $R_1$ | $R_2$ | $R_3$ | n' | $R_5$ | Salz | F. °C |
|---|---|---|---|---|---|---|---|
| 1 | H | H | H | 2 | H | 2HCl | 249 Zers.* |
| 2 | H | H | $CH_3$ | 2 | H | 2HCl | 216-219 |
| 3 | H | H | $CH_3$ | 2 | $OCH_3$ | 2HCl | 155 Zers. |
| 4 | H | H | $CH_3$ | 2 | F | Maleat | 202-204 |
| 5 | H | H | $CH_3$ | 2 | Cl | 2HCl | 203-206 |
| 6 | H | H | $CH_3$ | 2 | $CF_3$ | 2HCl | 180 Zers. |
| 7 | $CO_2C_2H_5$ | H | $CH_3$ | 2 | H | — | 138-141 |
| 8 | H | H | $(CH_2)_2OH$ | 2 | H | 2HCl | 210-212 |
| 9 | H | H | COOEt | 2 | H | — | 137-139 |
| 10 | H | H | $CH_3$ | 3 | H | Fumarat | 216-218 |
| 11 | H | H | $COOCH_2C_6H_5$ | 2 | H | — | NMR:5.2,3.5 |
| 12 | $CH_3$ | H | $CH_3$ | 2 | H | Maleat | 191-192.5 |
| 13 | $CH_3$ | $CH_3$ | $CH_3$ | 2 | H | | |

* (Zers. = Zersetzung).

Beispiel 9

Eine Lösung von 0,2 g 5-(4-Carboäthoxypiperazino)-imidazo-[2,1-b] [1,3,5] benzothiadiazepin in 2 ml trockenem Tetrahydrofuran wird auf einmal mit 100 mg Lithiumaluminiumhydrid versetzt und das Gemisch in einer Stickstoffatmosphäre 48 Stunden unter Rückfluss gekocht. Das Reaktionsgemisch wird auf Zimmertemperatur gekühlt, mit 0,2 ml 30 %-iger wässeriger Natriumhydroxydlösung gerührt und filtriert. Das Filtrat wird zur Trockene eingedampft und das Produkt gereinigt. Man erhält das 5-(4-Methylpiperazino)-imidazo [2,1-b] [1,3,5] benzothiadiazepin, welches bei 145-147° schmilzt und mit dem Produkt des Beispiels 1 identisch ist.

Beispiel 10

Eine Lösung von 88 mg 5-(4-Methylpiperazino)-imidazo-[2,1-b] [1,3,5] benzothiadiazepin in 1 ml Methylenchlorid wird bei 0° mit 74 mg m-Chlorperbenzoesäure versetzt. Das Gemisch wird über Nacht bei 0° gerührt, dann mit 1 ml Diäthyläther verdünnt und mit einem Aequivalent ätherischer Chlorwasserstoffsäurelösung versetzt. Der erhaltene Niederschlag wird abgetrennt und aus Methanol-Essigsäureäthylester umkristallisiert. Man erhält das 5-(4-Methyl-4-oxidopiperazino)-imidazo [2,1-b] [1,3,5] benzothiadiazepin-hydrochlorid, welches unter Zersetzung bei 155° schmilzt.

Beispiel 11

a) Eine Lösung von 0,5 g 5-(4-Carbobenzoxypiperazino)-imidazo [2,1-b] [1,3,5] benzothiadiazepin in 5 ml Methylenchlorid wird auf 0° gekühlt und mit einer Lösung von 0,26 g m-Chlorperbenzoesäure in 2 ml Methylenchlorid tropfenweise versetzt. Das Gemisch wird bei 0° 1,5 Stunden gerührt und das feste Material abfiltriert. Das Filtrat wird mit 10 %-iger wässeriger Kaliumcarbonatlösung und Wasser gewaschen, dann mit Magnesiumsulfat getrocknet und zur Trockene eingedampft. Man erhält das 5-(4-Carbobenzoxypiperazino)-imidazo [2,1-b] [1,3,5] benzothiadiazepin-1-oxid. Massenspektrum : m/e 435, 418, 387.

b) In analoger Weise, unter Verwendung von 0,61 g (2 Aequivalente) m-Chlorperbenzoesäure erhält man das 5-(4-Carbobenzoxypiperazino)-imidazo [2,1-b] [1,3,5] benzothiadiazepin-1,1-dioxid. Massenspektrum : m/e 451, 420, 406, 386.

Beispiel 12

a) Eine Lösung von 100 mg 5-(4-Carbobenzoxypiperazino)-imidazo [2,1-b] [1,3,5] benzothiadiazepin-1-oxid in 0,3 ml Essigsäure wird mit 0,35 ml 2-normaler Bromwasserstoffsäure in Essigsäure versetzt. Das Gemisch wird bei 100° eine Stunde gehalten und über Nacht bei Zimmertemperatur gerührt. Man gibt Diäthyläther dazu, filtriert das 5-(4H-Piperazino)-imidazo [2,1-b] [1,3,5]-benzothiadiazepin-hydrobromid ab und wäscht es mit Diäthyläther. Das Produkt schmilzt unter Zersetzung bei 75°.

b) In analoger Weise wird das 5-(4-Carbobenzoxypiperazino)-imidazo-[2,1-b] [1,3,5] benzothiadiazepin-1,1-dioxid in 5-(Piperazino)-imidazo-[2,1-b] [1,3,5] benzothiadiazepin-1,1-dioxid-hydrobromid umgewandelt. Rf = 0,353 (Silicagel, Essigsäureäthylester-Methylenchlorid, 1 : 1).

Beispiel 13

Ein Gemisch von 285 mg 5-(4H-Piperazino)-imidazo [2,1-b]-[1,3,5] benzothiadiazepin, 0,5 g Kaliumcarbonat, 0,142 g Methyljodid und 2 ml Aceton wird über Nacht gerührt und dann eingedampft. Der Rückstand wird mit Wasser versetzt und das Gemisch mit Methylenchlorid extrahiert. Der Extrakt wird über Magnesiumsulfat getrocknet, eingedampft und der Rückstand gereinigt. Man erhält das 5-(4-Methylpiperazino)-imidazo [2,1-b] [1,3,5] benzothiadiazepin, welches bei 145-147° schmilzt. Das Produkt ist mit demjenigen des Beispiels 1 identisch.

Beispiel 14

Herstellung von 10'000 Tabletten mit einem Gehalt von je 5 mg der aktiven Substanz :

| Bestandteile : | |
|---|---|
| 5-(4-Methylpiperazino)-imidazo [2,1-b] [1,3,5]-benzothiadiazepin-maleat | 50 g |
| Milchzucker | 1 157 g |
| Maisstärke | 75 g |
| Polyäthylenglykol 6000 | 75 g |
| Talkpulver | 75 g |
| Magnesiumstearat | 18 g |
| Gereinigte Wasser | q.s. |

Verfahren : Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

Beispiel 15

Herstellung von 10'000 Kapseln mit einem Gehalt von je 10 mg der aktiven Substanz :

Bestandteile :

| | |
|---|---|
| 5-[4-(2-Hydroxyäthyl)-piperazino]-imidazo [2,1-b] [1,3,5] benzothiadiazepindihydrochlorid | 100 g |
| Milchzucker | 1 800 g |
| Talkpulver | 100 g |

Verfahren : Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

In analoger Weise werden Tabletten oder Kapseln von anderen erfindungsgemässen Verbindungen hergestellt.

**Patentansprüche** (für die Vertragsstaaten : BE, CH, DE, FR, IT, LI, LU, NL, SE)

1. Verbindungen der allgemeinen Formel I

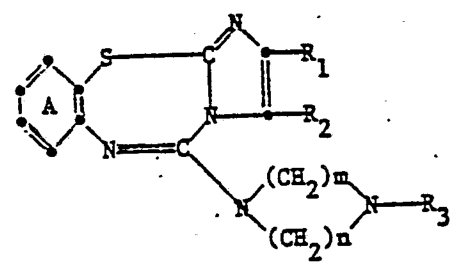

(I)

worin jedes der Symbole $R_1$ und $R_2$ Wasserstoff, $C_{1-7}$-Alkyl, $C_{1-7}$-Alkanoyl, Halogen, Cyan, Carboxy, Carbo-($C_{1-7}$)-alkoxy, Carbamoyl, Sulfamoyl, Mono- oder Di-($C_{1-7}$)-alkyl-(carbamoyl oder sulfamoyl) bedeutet, der Rest A unsubstituiert oder einfach durch $C_{1-7}$-Alkyl, $C_{1-7}$-Alkoxy, $C_{1-7}$-Alkylthio, Halogen, Trifluormethyl, Sulfamoyl, Mono- oder Di-($C_{1-7}$)-alkylsulfamoyl substituiert ist, jedes der Symbole m und n die ganze Zahl 2 oder 3 bedeutet, und $R_3$ für Wasserstoff, $C_{1-7}$-Alkyl, $C_{1-7}$-Alkanoyl, $C_{1-7}$-Alkoxycarbonyl, Phenyl-($C_{1-7}$)-alkoxycarbonyl oder Hydroxy-($C_{1-7}$)-alkyl steht, worin Hydroxy vom Stickstoffatom durch mindestens 2 Kohlenstoffatome getrennt ist, sowie, soweit $R_3$ für $C_{1-7}$-Alkyl oder Hydroxy-($C_{1-7}$)-alkyl steht, entsprechende N-Oxide am N-$R_3$ von Verbindungen der Formel I, und/oder ihre S-Oxide, und die Salze von solchen Verbindungen.

2. Verbindungen nach Anspruch 1 der Formel II

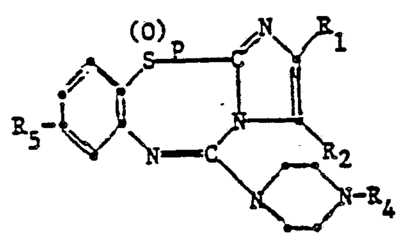

(II)

worin jedes der Symbole $R_1$ und $R_2$ Wasserstoff oder $C_{1-7}$-Alkyl bedeutet, $R_4$ für Wasserstoff, $C_{1-7}$-Alkyl oder 2- oder 3-Hydroxy-($C_{1-7}$)-alkyl steht, $R_5$ Wasserstoff, $C_{1-7}$-Alkyl, $C_{1-7}$-Alkoxy, Halogen, oder Trifluormethyl bedeutet, und p für die Zahl von 0 bis 2 steht, sowie N-Oxide am $NR_4$ soweit $R_4$ für $C_{1-7}$-Alkyl steht und Salze von solchen Verbindungen.

3. 5-(4-Methylpiperazino)-imidazo [2,1-b] [1,3,5] benzothiadiazepin und seine Salze.

4. 8-Chlor-5-(4-methylpiperazino)-imidazo [2,1-b] [1,3,5] benzothiadiazepin und seine Salze.

5. 3-Methyl-5-(4-methylpiperazino)-imidazo [2,1-b] [1,3,5] benzothiadiazepin und seine Salze.

6. 5-[4-(2-Hydroxyäthyl)-piperazino]-imidazo [2,1-b] [1,3,5] benzothiadiazepin und seine Salze.

7. Pharmazeutische Präparate enthaltend Verbindungen gemäss Anspruch 1, zusammen mit einem pharmazeutischen Trägermaterial.

8. Die im Anspruch 1 genannten Verbindungen zur Anwendung in einem Verfahren zur therapeutischen Behandlung des menschlichen oder tierischen Körpers.

9. Die im Anspruch 1 genannten Verbindungen zur Anwendung in einem Mittel zur Anwendung in einem Verfahren zur therapeutischen Behandlung des menschlichen oder tierischen Körpers.

10. Verwendung der im Anspruch 1 genannten Verbindungen zur Herstellung von pharmazeutischen Präparaten.

11. Verfahren zur Herstellung von den im Anspruch 1 genannten Verbindungen, dadurch gekennzeichnet, dass man

a) Verbindungen der allgemeinen Formel III oder ihre Salze mit Verbindungen der allgemeinen Formel IV kondensiert

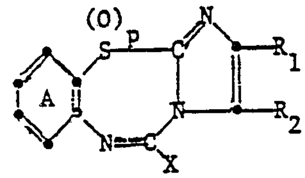 (III)

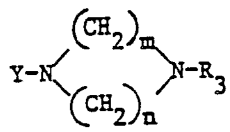 (IV)

worin X Halogen, Niederalkoxy, Niederalkylthio, Cyanato oder Thiocyanato bedeutet, Y für Wasserstoff oder ein Alkalimetallatom steht, p eine Zahl von Null bis 2 bedeutet, und die anderen Symbole die für die Formel I im Anspruch 1 angegebenen Bedeutungen haben, oder

b) Verbindungen der allgemeinen Formel V

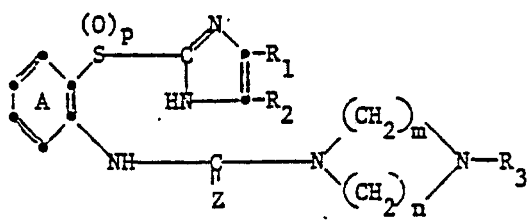 (V)

worin Z ein Sauerstoff- oder Schwefelatom oder NH bedeutet, und die anderen Symbole die oben angegebenen Bedeutungen haben, unter entwässernden oder dehydrosulfurierenden Bedingungen ringschliesst, und, wenn eine Verbindung der Formel I erwünscht ist, worin $R_3$ $C_{1-7}$-Alkyl bedeutet, in eine Verbindung der Formel I, worin $R_3$ Wasserstoff oder ein Alkalimetallatom bedeutet, $C_{1-7}$-Alkyl einführt, und/oder, wenn eine Verbindung der Formel I erwünscht ist, worin $R_3$ Wasserstoff bedeutet, eine erhaltene Verbindung, worin $R_3$ einen Acylrest bedeutet, hydrolisiert, und/oder wenn eine Verbindung der Formel I erwünscht ist, worin $R_3$ Methyl bedeutet, in einem erhaltenen Produkt, worin $R_3$ $C_{1-7}$-Alkoxycarbonyl oder Phenyl-($C_{1-7}$)-alkoxycarbonyl bedeutet, diese Reste zu Methyl reduziert, und/oder, wenn ein N- und/oder S-Oxid erwünscht ist, eine erhaltene Verbindung, worin $R_3$ sich von Wasserstoff unterscheidet, oxidiert, und/oder, wenn lediglich ein N-Oxid erwünscht ist, eine erhaltene Verbindung, worin $R_3$ sich von Wasserstoff unterscheidet, spezifisch oxidiert, und/oder, wenn lediglich ein S-Oxid erwünscht ist, eine erhaltene Verbindung, worin $R_3$ einen Acylrest bedeutet, S-oxidiert und gegebenenfalls den Acylrest abspaltet, und/oder, wenn erwünscht, eine erhaltene freie Verbindung der Formel I in ein Salz oder ein erhaltenes Salz in die freie Verbindung der Formel I oder in ein anderes Salz überführt, und/oder, wenn erwünscht, ein erhaltenes Gemisch von Isomeren oder Razematen in die einzelnen Isomere oder Razematen auftrennt, und/oder, wenn erwünscht, erhaltene Razemate in die optischen Antipoden aufspaltet.

12. Die nach dem Verfahren des Anspruchs 11 erhältlichen Verbindungen.

**Patentansprüche** (für den Vertragsstaat AT)

1. Verfahren zur Herstellung von 5-Diazocycloalkyl-imidazo-[2,1-b] [1,3,5] benzothiadiazepinen der allgemeinen Formel I

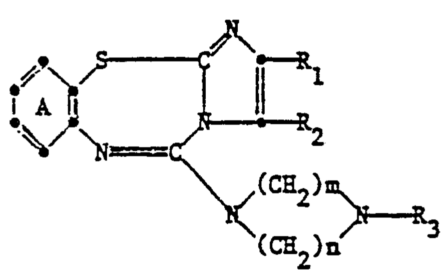 (I)

worin jedes der Symbole $R_1$ und $R_2$ Wasserstoff, $C_{1-7}$-Alkyl, $C_{1-7}$-Alkanoyl, Halogen, Cyan, Carboxy, Carbo-($C_{1-7}$)-alkoxy, Carbamoyl, Sulfamoyl, Mono- oder Di-($C_{1-7}$)-alkyl-(carbamoyl oder sulfamoyl) bedeutet, der Rest A unsubstituiert oder einfach durch $C_{1-7}$-Alkyl, $C_{1-7}$-Alkoxy, $C_{1-7}$-Alkylthio, Halogen, Trifluormethyl, Sulfamoyl, Mono- oder Di-($C_{1-7}$)-alkylsulfamoyl substituiert ist, jedes der Symbole m und n die ganze Zahl 2 oder 3 bedeutet, und $R_3$ für Wasserstoff, $C_{1-7}$-Alkyl, $C_{1-7}$-Alkanoyl, $C_{1-7}$-Alkoxycarbonyl, Phenyl-($C_{1-7}$)-alkoxycarbonyl oder Hydroxy-($C_{1-7}$)-alkyl steht, worin Hydroxy vom Stickstoffatom durch mindestens 2 Kohlenstoffatome getrennt ist, sowie, soweit $R_3$ für $C_{1-7}$-Alkyl oder Hydroxy-($C_{1-7}$)-alkyl steht, entsprechenden N-Oxiden am N-$R_3$ von Verbindungen der Formel I, und/oder ihren S-Oxiden und Salzen von solchen Verbindungen, dadurch gekennzeichnet, dass man

a) Verbindungen der allgemeinen Formel III oder ihre Salze mit Verbindungen der allgemeinen Formel IV kondensiert

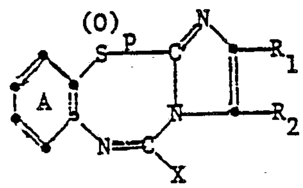 (III) 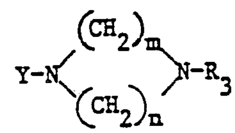 (IV)

worin X Halogen, Niederalkoxy, Niederalkylthio, Cyanato oder Thiocyanato bedeutet, Y für Wasserstoff oder ein Alkalimetallatom steht, p eine Zahl von Null bis 2 bedeutet, und die anderen Symbole die für die Formel I angegebenen Bedeutungen haben, oder

b) Verbindungen der allgemeinen Formel V

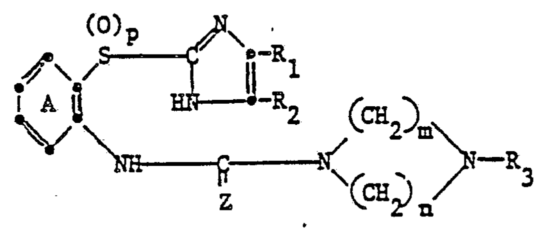 (V)

worin Z ein Sauerstoff- oder Schwefelatom oder NH bedeutet, und die anderen Symbole die oben angegebenen Bedeutungen haben, unter entwässernden oder dehydrosulfurierenden Bedingungen ringschliesst, und, wenn eine Verbindung der Formel I erwünscht ist, worin $R_3$ $C_{1-7}$-Alkyl bedeutet, in eine Verbindung der Formel I, worin $R_3$ Wasserstoff oder ein Alkalimetallatom bedeutet, $C_{1-7}$-Alkyl einführt, und/oder, wenn eine Verbindung der Formel I erwünscht ist, worin $R_3$ Wasserstoff bedeutet, eine erhaltene Verbindung, worin $R_3$ einen Acylrest bedeutet, hydrolisiert, und/oder, wenn eine Verbindung der Formel I erwünscht ist, worin $R_3$ Methyl bedeutet, in einer erhaltenen Verbindung, worin $R_3$ $C_{1-7}$-Alkoxycarbonyl oder Phenyl-($C_{1-7}$)-alkoxycarbonyl bedeutet, diese Reste zu Methyl reduziert, und/oder, wenn ein N- und/oder S-Oxid erwünscht ist, eine erhaltene Verbindung, worin $R_3$ sich von Wasserstoff unterscheidet, oxidiert, und/oder, wenn lediglich ein N-Oxid erwünscht ist, eine erhaltene Verbindung, worin $R_3$ sich von Wasserstoff unterscheidet, spezifisch oxidiert, und/oder, wenn lediglich ein S-Oxid erwünscht ist, eine erhaltene Verbindung, worin $R_3$ einen Acylrest bedeutet, S-oxidiert und gegebenenfalls den Acylrest abspaltet, und/oder, wenn erwünscht, eine erhaltene freie Verbindung der Formel I in ein Salz oder ein erhaltenes Salz in die freie Verbindung der Formel I oder in ein anderes Salz überführt, und/oder, wenn erwünscht, ein erhaltenes Gemisch von Isomeren oder Razematen in die einzelnen Isomere oder Razematen auftrennt, und/oder, wenn erwünscht, erhaltene Razemate in die optischen Antipoden aufspaltet.

2. Verfahren nach Anspruch 1, dadurch gekennzeichnet, dass man Verbindungen der Formel II

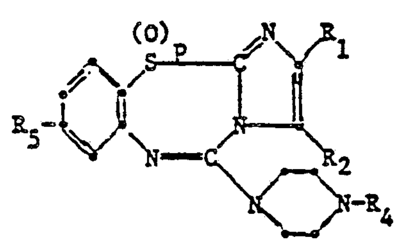 (II)

worin jedes der Symbole $R_1$ und $R_2$ Wasserstoff oder $C_{1-7}$-Alkyl bedeutet, $R_4$ für Wasserstoff, $C_{1-7}$-Alkyl oder 2- oder 3-Hydroxy-($C_{1-7}$)-alkyl steht, $R_5$ Wasserstoff, $C_{1-7}$-Alkyl, $C_{1-7}$-Alkoxy Halogen, oder Trifluormethyl bedeutet, und p für die Zahl von 0 bis 2 steht, sowie N-Oxide am $NR_4$ soweit $R_4$ für $C_{1-7}$-Alkyl steht und Salze von solchen Verbindungen herstellt.

3. Verfahren nach Anspruch 1, dadurch gekennzeichnet, dass man 5-(4-Methylpiperazino)-imidazo [2,1-b] [1,3,5] benzothiadiazepin, 8-Chlor-5-(4-methylpiperazino)-imidazo [2,1-b] [1,3,5]-benzothiadiazepin, 3-Methyl-5-(4-methylpiperazino)-imidazo [2,1-b] [1,3,5] benzothiadiazepin und 5-[4-(2-Hydroxyäthyl)-piperazino]-imidazo [2,1-b]-[1,3,5] benzothiadiazepin und ihre Salze herstellt.

4. Verfahren nach Anspruch 1 zur Herstellung von 5-Diazacycloalkyl-imidazo [2,1-b] [1,3,5] benzothiadiazepinen der im Anspruch 1 angegebenen Formel I, worin $R_3$ Wasserstoff, $C_{1-7}$-Alkyl, $C_{1-7}$-Alkanoyl, $C_{1-7}$-Alkoxycarbonyl oder Hydroxy-($C_{1-7}$)-alkyl bedeutet, worin die Hydroxygruppe vom Stickstoffatom durch mindestens 2 Kohlenstoffatome getrennt ist, und die anderen Symbole die im Anspruch 1 angegebene Bedeutung haben ; ihren N- und/oder S-Oxiden gemäss Anspruch 1, und Salzen, dadurch gekennzeichnet, dass man

a) Verbindungen der allgemeinen Formel III oder ihre Salze mit Verbindungen der allgemeinen Formel IV kondensiert

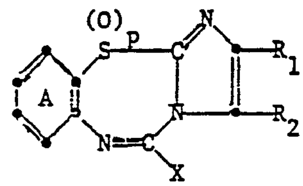 (III) 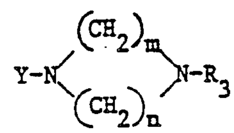 (IV)

worin p für Null steht, X Halogen, Niederalkoxy, Niederalkylthio, Cyanato oder Thiocyanato bedeutet, Y für Wasserstoff oder ein Alkylimetallatom steht, jedes der Symbole $R_1$ und $R_2$ Wasserstoff, $C_{1-7}$-Alkyl, $C_{1-7}$-Alkanoyl, Halogen, Cyan, Carbo-($C_{1-7}$)-alkoxy, Carbamoyl, Sulfamoyl, Mono- oder Di-($C_{1-7}$)-alkyl-(carbamoyl oder sulfamoyl bedeutet, und die anderen Symbole die oben für die Formel I angegebene Bedeutung haben, oder

b) Verbindungen der allgemeinen Formel V

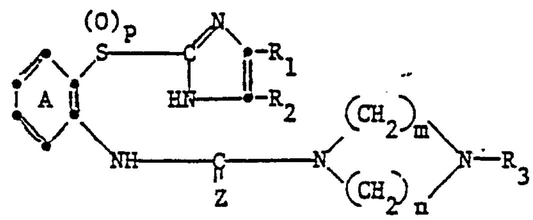 (V)

worin p für Null steht, Z ein Sauerstoff- oder Schwefelatom oder·NH bedeutet, und die anderen Symbole die oben angegebenen Bedeutungen haben, unter entwässernden oder dehydrosulfurierenden Bedingungen ringschliesst, und, wenn eine Verbindung der Formel I erwünscht ist, worin $R_3$ $C_{1-7}$-Alkyl oder Hydroxy-($C_{1-7}$)-alkyl bedeutet, in eine Verbindung der Formel I, worin $R_3$ Wasserstoff oder ein Alkalimetall bedeutet, einen genannten Rest durch Umsetzung mit einem reaktionsfähigen Ester eines Niederalkanols oder eines mono-veresterten Niederalkandiols einführt, und/oder, wenn eine Verbindung der Formel I erwünscht ist, worin $R_3$ einen oben definierten Acylrest bedeutet, eine erhaltene Verbindung, worin $R_3$ für Wasserstoff steht, durch Umsetzung mit reaktionsfähigen Säurederivaten entsprechender Säuren, acyliert, und/oder, wenn ein N- und/oder S-Oxid erwünscht ist, eine erhaltene Verbindung, worin $R_3$ sich von Wasserstoff unterscheided, oxidiert, und/oder, wenn lediglich ein N-Oxid erwünscht ist, eine erhaltene Verbindung, worin $R_3$ sich von Wasserstoff unterscheided, spezifisch oxidiert, und/oder, wenn erwünscht, eine erhaltene freie Verbindung der Formel I in ein Salz oder ein erhaltenes Salz in die freie Verbindung der Formel I oder in ein anderes Salz überführt, und/oder, wenn erwünscht, ein erhaltenes Geschmisch von Isomeren oder Razematen in die einzelnen Isomeren oder Razemate auftrennt, und/oder, wenn erwünscht, erhaltene Razemate in die optischen Antipoden aufspaltet.

5. Verfahren nach Anspruch 4, dadurch gekennzeichnet, dass man Verbindungen der im Anspruch 1 angegebenen Formel I, worin $R_3$ Wasserstoff, $C_{1-7}$-Alkyl, $C_{1-7}$-Alkanoyl, $C_{1-7}$-Alkoxycarbonyl oder Hydroxy-($C_{1-7}$)-alkyl bedeutet, worin die Hydroxygruppe vom Stickstoffatom durch mindestens 2 Kohlenstoffatome getrennt ist, und die anderen Symbole die im Anspruch 2 angegebene Bedeutung haben ; ihre N- und/oder S-Oxide und Salze, herstellt.

6. Verfahren nach Anspruch 4, dadurch gekennzeichnet, dass man Verbindungen der allgemeinen Formel II

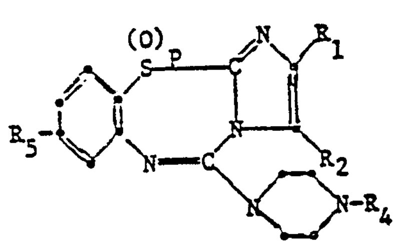 (II)

worin jedes der Symbole $R_1$ und $R_2$ Wasserstoff bedeutet, $R_4$ für Wasserstoff, $C_{1-7}$-Alkyl oder 2- oder 3-Hydroxy-($C_{1-7}$)-alkyl steht, $R_5$ Wasserstoff, $C_{1-7}$-Alkyl, $C_{1-7}$-Alkoxy, Halogen oder Trifluormethyl bedeutet, und p für die Zahl Null steht, und Salze dieser Verbindungen herstellt.

7. Verfahren nach Anspruch 4, dadurch gekennzeichnet, dass man 5-(4-Methylpiperazino)-imidazo [2,1-b] [1,3,5] benzothiadiazepin, 8-Chlor-5-(4-methylpiperazino)-imidazo [2,1-b] [1,3,5] benzothiadiazepin und 5-[4-(2-Hydroxyäthyl)-piperazino]-imidazo [2,1-b] [1,3,5] benzothiadiazepin und ihre Salze herstellt.

8. Verfahren zur Herstellung eines pharmazeutischen Präparates, gekennzeichnet durch die Verarbeitung eines erfindungsgemässen Wirkstoffes nach einem der Ansprüche 1 bis 3, mit einem pharmazeutischen Trägermaterial.

9. Verfahren zur Herstellung eines pharmazeutischen Präparates, gekennzeichnet durch die Verarbeitung eines erfindungsgemässen Wirkstoffes nach einem der Ansprüche 4 bis 7 mit einem pharmazeutischen Trägermaterial.

**Claims** (for the Contracting States : DE, FR, CH, LI, IT, NL, BE, SE, LU)

1. A compound of the general formula I

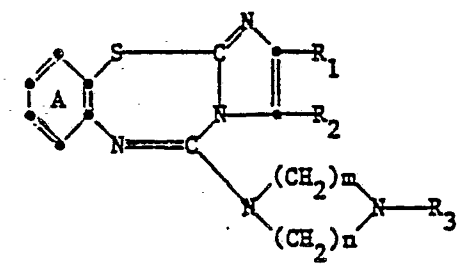

(I)

wherein each of $R_1$ and $R_2$ is hydrogen, $C_1$-$C_7$-alkyl, $C_1$-$C_7$-alkanoyl, halogen, cyano, carboxy, carboalkoxy containing 1 to 7 carbon atoms in the alkoxy moiety, carbamoyl, sulfamoyl, or is mono- or dialkylcarbamoyl or mono- or dialkylsulfamoyl, each containing 1 to 7 carbon atoms in the alkyl moiety or moieties, the radical A is unsubstituted or monosubstituted by $C_1$-$C_7$-alkyl, $C_1$-$C_7$-alkoxy, $C_1$-$C_7$-alkylthio, halogen, trifluoromethyl, sulfamoyl, or by mono- or dialkylsulfamoyl containing 1 to 7 carbon atoms in the alkyl moiety or moieties, each of m and n is the integer 2 or 3, and $R_3$ is hydrogen, $C_1$-$C_7$-alkyl, $C_1$-$C_7$-alkanoyl, $C_1$-$C_7$-alkoxycarbonyl, phenylalkoxycarbonyl containing 1 to 7 carbon atoms in the alkoxy moiety or hydroxyalkyl containing 1 to 7 carbon atoms in the alkyl moiety and wherein the hydroxyl group is separated from the nitrogen atom by at least 2 carbon atoms, or, provided $R_3$ is $C_1$-$C_7$-alkyl or hydroxyalkyl containing 1 to 7 carbon atoms in the alkyl moiety, a corresponding N oxide at the N-$R_3$ radical of a compound of formula I, and/or an S oxide or a salt thereof.

2. A compound according to claim 1 of formula II

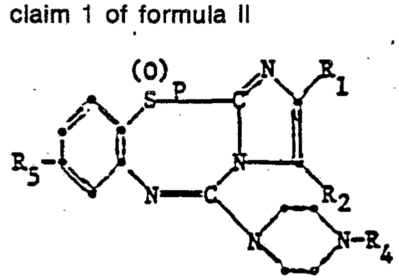

(II)

wherein each of $R_1$ and $R_2$ is hydrogen or $C_1$-$C_7$-alkyl, $R_4$ is hydrogen, $C_1$-$C_7$-alkyl, or is 2-hydroxyalkyl or 3-hydroxylalkyl, each containing 1 to 7 carbon in the alkyl moiety, $R_5$ is hydrogen, $C_1$-$C_7$-alkyl, $C_1$-$C_7$-alkoxy, halogen, or trifluoromethyl, and p is a value from 0 to 2, or, provided $R_4$ is $C_1$-$C_7$-alkyl, an N oxide at the $NR_4$ radical, or a salt thereof.

3. 5-(4-Methylpiperazino)imidazo [2,1-b] [1,3,5] benzothiadiazepine, or a salt thereof.

4. 8-Chloro-5-(4-methylpiperazino)imidazo [2,1-b] [1,3,5]-benzothiadiazepine, or a salt thereof.

5. 3-Methyl-5-(4-methylpiperazino)imidazo [2,1-b] [1,3,5]-benzothiadiazepine, or a salt thereof.

6. 5-[4-(2-Hydroxyethyl)piperazino)imidazo [2,1-b] [1,3,5]-benzothiadiazepine, or a salt thereof.

7. A pharmaceutical composition containing a compound according to claim 1, together with a pharmaceutical carrier.

8. A compound according to claim 1 for use in a therapeutic method of treating the human or animal body.

9. A compound according to claim 1 for use in a composition for use in a therapeutic method of treating the human of animal body.

10. Use of a compound according to claim 1 for the preparation of a pharmaceutical composition.

11. A process for the preparation of a compound according to claim 1, which process comprises

a) condensing a compound of the general formula III, or a salt thereof, with a compound of the general formula IV

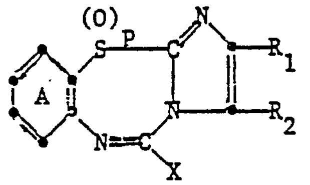
(III)

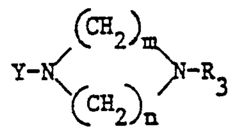
(IV)

in which formulae X is halogen, lower alkoxy, lower alkylthio, cyanato or thiocyanato, Y is hydrogen or an alkali metal atom, p is a value from 0 to 2, and the other symbols are as defined for formula I in claim 1, or

b) ring-closing a compound of the general formula V

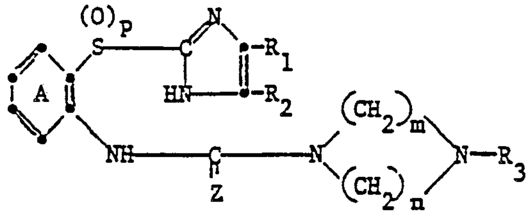
(V)

wherein Z is an oxygen or sulfur atom or NH, and the other symbols are as defined above, under conditions of dewatering or dehydrosulfuration, and, if a compound of formula I is desired, wherein $R_3$ is $C_1$-$C_7$-alkyl, introducing $C_1$-$C_7$-alkyl into a compound of formula I, wherein $R_3$ is hydrogen or an alkali metal atom, and/or, if a compound of formula I is desired, wherein $R_3$ is hydrogen, hydrogenating a resultant compound, wherein $R_3$ is an acyl radical, and/or, if a compound of formula I is desired, wherein $R_3$ is methyl, in a resultant product, wherein $R_3$ is $C_1$-$C_7$-alkoxycarbonyl or phenylalkoxycarbonyl containing 1 to 7 carbon atoms in the alkoxy moiety, reducing said radicals to methyl, and/or, if an N and/or S oxide is desired, oxidising a resultant compound, wherein $R_3$ is other than hydrogen, and/or, if only an N oxide is desired, specifically oxidising a resultant compound, wherein $R_3$ is other than hydrogen, and/or, if only an S oxide is desired, S-oxidising a resultant compound, wherein $R_3$ is an acyl radical, and optionally removing the acyl radical, and/or, if desired, converting a resultant free compound of formula I into a salt or converting a resultant salt into the free compound of formula I or into another salt, and/or, if desired, separating a resultant mixture of isomers or racemates into the individual isomers or racemates, and/or, if desired, splitting the resultant racemates into the optical antipodes.

12. A compound obtainable by the process according to claim 11.

**Claims** (for the Contracting State AT)

1. A process for the preparation of a 5-diazocycloalkylimidazo [2,1-b] [1,3,5] benzothiadiazepine of the general formula

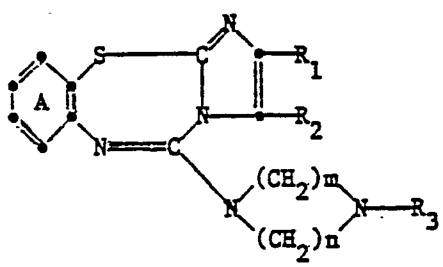
(I)

wherein each of $R_1$ and $R_2$ is hydrogen, $C_1$-$C_7$-alkyl, $C_1$-$C_7$-alkanoyl, halogen, cyano, carboxy, carboalkoxy containing 1 to 7 carbon atoms in the alkoxy moiety, carbamoyl, sulfamoyl, or is mono- or dialkylcarbamoyl or mono- or dialkylsulfamoyl, each containing 1 to 7 carbon atoms in the alkyl moiety or moieties, the radical A is unsubstituted or monosubstituted by $C_1$-$C_7$-alkyl, $C_1$-$C_7$-alkoxy, $C_1$-$C_7$-alkylthio, halogen, trifluoromethyl, sulfamoyl, or by mono- or dialkylsulfamoyl containing 1 to 7 carbon atoms in the alkyl moiety or moieties, each of m and n is the integer 2 or 3, and $R_3$ is hydrogen, $C_1$-$C_7$-alkyl, $C_1$-$C_7$-alkanoyl, $C_1$-$C_7$-alkoxycarbonyl, phenylalkoxycarbonyl containing 1 to 7 carbon atoms in the alkoxy moiety or hydroxyalkyl containing 1 to 7 carbon atoms in the alkyl moiety and wherein the hydroxyl group is separated from the nitrogen atom by at least 2 carbon atoms, or, provided $R_3$ is $C_1$-$C_7$-alkyl or hydroxyalkyl containing 1 to 7 carbon atoms in the alkyl moiety, a corresponding N oxide at the N-$R_3$

radical of a compound of formula I, and/or an S oxide or a salt thereof, which process comprises

a) condensing a compound of the general formula III, or a salt thereof, with a compound of the general formula IV

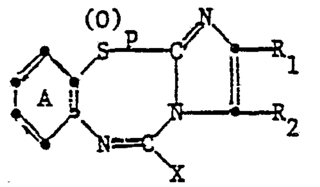

(III)

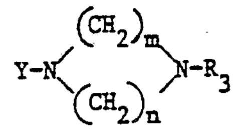

(IV)

in which formulae X is halogen, lower alkoxy, lower alkylthio, cyanato or thiocyanato, Y is hydrogen or an alkali metal atom, p is a value from 0 to 2, and the other symbols are as defined for formula I, or

b) ring-closing a compound of the general formula V

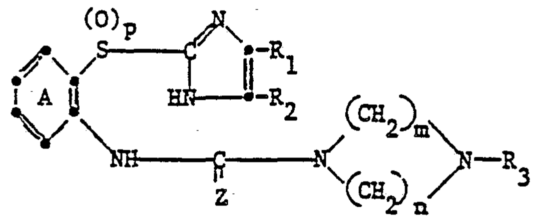

(V)

wherein Z is an oxygen or sulfur atom or NH, and the other symbols are as defined above, under conditions of dewatering or dehydrosulfuration, and, if a compound of formula I is desired, wherein $R_3$ is $C_1$-$C_7$-alkyl, introducing $C_1$-$C_7$-alkyl into a compound of formula I, wherein $R_3$ is hydrogen or an alkali metal atom, and/or, if a compound of formula I is desired, wherein $R_3$ is hydrogen, hydrogenating a resultant compound, wherein $R_3$ is an acyl radical, and/or, if a compound of formula I is desired, wherein $R_3$ is methyl, in a resultant product, wherein $R_3$ is $C_1$-$C_7$-alkoxycarbonyl or phenylalkoxycarbonyl containing 1 to 7 carbon atoms in the alkoxy moiety, reducing said radicals to methyl, and/or, if an N and/or S oxide is desired, oxidising a resultant compound, wherein $R_3$ is other than hydrogen, and/or, if only an N oxide is desired, specifically oxidising a resultant compound, wherein $R_3$ is other than hydrogen, and/or, if only an S oxide is desired, S-oxidising a resultant compound, wherein $R_3$ is an acyl radical, and optionally removing the acyl radical, and/or, if desired, converting a resultant free compound of formula I into a salt or converting a resultant salt into the free compound of formula I or into another salt, and/or, if desired, separating a resultant mixture of isomers or racemates into the individual isomers or racemates, and/or, if desired, splitting the resultant racemates into the optical antipodes.

2. A process according to claim 1, which process comprises preparing a compound of formula II

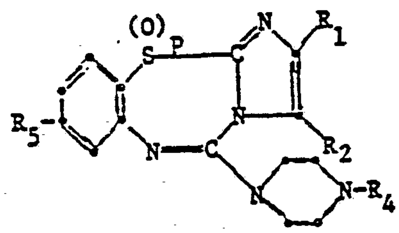

(II)

wherein each of $R_1$ and $R_2$ is hydrogen or $C_1$-$C_7$-alkyl, $R_4$ is hydrogen, $C_1$-$C_7$-alkyl, or is 2-hydroxyalkyl or 3-hydroxylalkyl, each containing 1 to 7 carbon atoms in the alkyl moiety, $R_5$ is hydrogen, $C_1$-$C_7$-alkyl, $C_1$-$C_7$-alkoxy, halogen, or trifluoromethyl, and p is a value from 0 to 2, or, provided $R_4$ is $C_1$-$C_7$-alkyl, an N oxide at the $NR_4$ radical, or a salt thereof.

3. A process according to claim 1, which process comprises preparing a compound selected from the series consisting of 5-(4-methylpiperazino)imidazo [2,1-b] [1,3,5] benzothiadiazepine, 8-chloro-5-(4-methylpiperazino)imidazo [2,1-b] [1,3,5] benzothiadiazepine, 3-methyl-5-(4-methylpiperazino)imidazo [2,1-b] [1,3,5] benzothiadiazepine and 5-[4-(2-hydroxyethyl)piperazino)-imidazo [2,1-b] [1,3,5] benzothiadiazepine, or a salt thereof.

4. A process according to claim 1 for the preparation of a 5-diazacycloalkylimidazo [2,1-b] [1,3,5] benzothiadiazepine of formula I according to claim 1, wherein $R_3$ is hydrogen, $C_1$-$C_7$-alkyl, $C_1$-$C_7$-alkanoyl, $C_1$-$C_7$-alkoxycarbonyl or hydroxyalkyl containing 1 to 7 carbon atoms in the alkyl moiety and wherein the hydroxyl group is separated from the nitrogen atom by at least 2 carbon atoms, and the other symbols are as defined in claim 1 ; or an N and/or S oxide thereof according to claim 1, or a salt thereof, which process comprises

a) condensing a compound of the general formula III, or a salt thereof, with a compound of the general formula IV

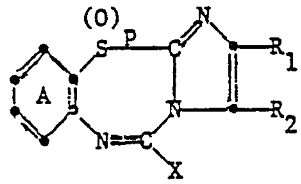 (III) 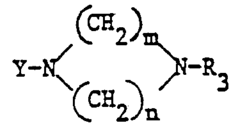 (IV)

in which formula p is zero, X is halogen, lower alkoxy, lower alkylthio, cyanato or thiocyanato, Y is hydrogen or an alkali metal atom, each of $R_1$ and $R_2$ is hydrogen, $C_1$-$C_7$-alkyl, $C_1$-$C_7$-alkanoyl, halogen, cyano, carboalkoxy containing 1 to 7 carbon atoms in the alkoxy moiety, carbamoyl, sulfamoyl, or is mono- or dialkylcarbamoyl or mono- or dialkylsulfamoyl, each containing 1 to 7 carbon atoms in the alkyl moiety or moieties, and the other symbols are as defined above for formula I, or

b) ring-closing a compound of the general formula V

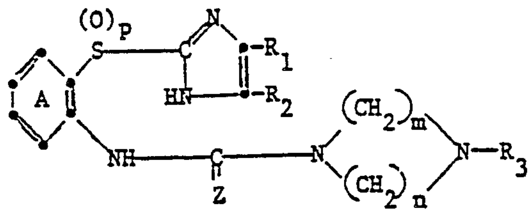

(V)

wherein p is zero, Z is an oxygen or sulfur atom or NH, and the other symbols are as defined above, under conditions of dewatering or dehydrosulfuration, and, if a compound of formula I is desired, wherein $R_3$ is $C_1$-$C_7$-alkyl or hydroxyalkyl containing 1 to 7 carbon atoms in the alkyl moiety, introducing one of said radicals into a compound of formula I, wherein $R_3$ is hydrogen or an alkali metal, by reaction with a reactive ester of a lower alkanol or of a monoesterified lower alkanediol, and/or, if a compound of formula I is desired, wherein $R_3$ is an acyl radical as defined above, acylating a resultant compound, wherein $R_3$ is hydrogen, by reaction with a reactive acid derivative of a corresponding acid, and/or, if an N and/or S oxide is desired, oxidising a resultant compound, wherein $R_3$ is other than hydrogen, and/or, if only an N oxide is desired, specifically oxidising a resultant compound, wherein $R_3$ is other than hydrogen, and/or, if desired, converting a resultant free compound of formula I into a salt or converting a resultant salt into the free compound of formula I or into another salt, and/or, if desired, separating a resultant mixture of isomers or racemates into the individual isomers or racemates, and/or, if desired, splitting the resultant racemates into the optical antipodes.

5. A process according to claim 4, which process comprises preparing a compound of formula I according to claim 1, wherein $R_3$ is hydrogen, $C_1$-$C_7$-alkyl, $C_1$-$C_7$-alkanoyl, $C_1$-$C_7$-alkoxycarbonyl or hydroxyalkyl containing 1 to 7 carbon atoms in the alkyl moiety and wherein the hydroxyl group is separated from the nitrogen atom by at least 2 carbon atoms, and the other symbols are as defined in claim 2 ; or an N and/or S oxide or a salt thereof.

6. A process according to claim 4, which process comprises preparing a compound of the general formula II

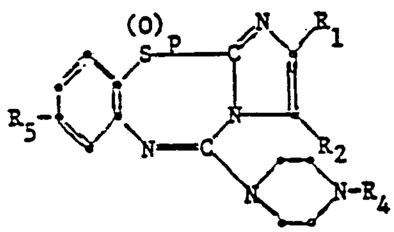

(II)

wherein each of $R_1$ and $R_2$ is hydrogen, $R_4$ is hydrogen, $C_1$-$C_7$-alkyl, or is 2-hydroxyalkyl or 3-hydroxyalkyl, each containing 1 to 7 carbon atoms in the alkyl moiety, $R_5$ is hydrogen, $C_1$-$C_7$-alkyl, $C_1$-$C_7$-alkoxy, halogen or trifluoromethyl, and p is zero, or a salt thereof.

7. A process according to claim 4, which process comprises preparing a compound selected from the series consisting of 5-(4-methylpiperazino)imidazo [2,1-b] [1,3,5] benzothiadiazepine, 8-chloro-5-(4-methylpiperazino)imidazo [2,1-b] [1,3,5] benzothiadiazepine and 5-[4-(2-hydroxyethyl) piperazino]-imidazo [2,1-b] [1,3,5] benzothiadiazepine, or a salt thereof.

8. A process for the preparation of a pharmaceutical composition, which process comprises combining a compound of the invention according to any of claims 1 to 3 with a pharmaceutical carrier.

9. A process for the preparation of a pharmaceutical composition, which process comprises combining a compound of the invention according to any one of claims 4 to 7 with a pharmaceutical carrier.

**Revendications** (pour les Etats contractants : DE, FR, CH, LI, IT, NL, BE, SE, LU)

1. Composés de formule générale I

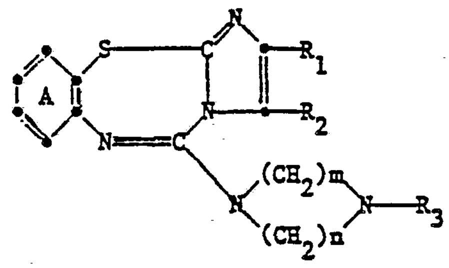

(I)

dans laquelle chacun des symboles $R_1$ et $R_2$ représente l'hydrogène, un groupe alkyle en $C_1$-$C_7$, alcanoyle en $C_1$-$C_7$, un halogène, un groupe cyano, carboxy, carbo-(alcoxy en $C_1$-$C_7$), carbamoyle, sulfamoyle, mono- ou di-(alkyle en $C_1$-$C_7$)-(carbamoyle ou sulfamoyle), le reste A est non substitué ou monosubstitué par un groupe alkyle en $C_1$-$C_7$, alcoxy en $C_1$-$C_7$, alkylthio en $C_1$-$C_7$, un halogène, un groupe trifluorométhyle, sulfamoyle, mono- ou di-(alkyle en $C_1$-$C_7$)-sulfamoyle, chacun des symboles m et n représente le nombre entier 2 ou 3 et $R_3$ représente l'hydrogène, un groupe alkyle en $C_1$-$C_7$, alcanoyle en $C_1$-$C_7$, (alcoxy en $C_1$-$C_7$)-carbonyle, phényl-(alcoxy en $C_1$-$C_7$)-carbonyle ou hydroxyalkyle en $C_1$-$C_7$, le groupe hydroxy étant séparé de l'atome d'azote par au moins deux atomes de carbone, et, lorsque $R_3$ représente un groupe alkyle en $C_1$-$C_7$ ou hydroxyalkyle en $C_1$-$C_7$, les N-oxydes correspondants en N-$R_3$ des composés de formule I, et/ou leurs S-oxydes et les sels de ces composés.

2. Composés selon la revendication 1, répondant à la formule II

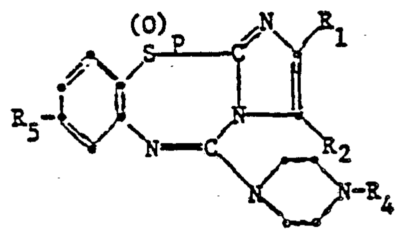

(II)

dans laquelle chacun des symboles $R_1$ et $R_2$ représente l'hydrogène ou un groupe alkyle en $C_1$-$C_7$, $R_4$ représente l'hydrogène, un groupe alkyle en $C_1$-$C_7$ ou 2- ou 3-hydroxyalkyle en $C_1$-$C_7$, $R_5$ représente l'hydrogène, un groupe alkyle en $C_1$-$C_7$, alcoxy en $C_1$-$C_7$, un halogène ou un groupe trifluorométhyle, et p est un nombre de 0 à 2, ainsi que les N-oxydes en $NR_4$ lorsque $R_4$ représente un groupe alkyle en $C_1$-$C_7$, et les sels de ces composés.

3. La 5-(4-méthylpipérazino)-imidazo [2,1-b] [1,3,5] benzothiadiazépine et ses sels.

4. La 8-chloro-5-(4-méthylpipérazino)-imidazo [2,1-b] [1,3,5] benzothiadiazépine et ses sels.

5. La 3-méthyl-5-(4-méthylpipérazino)-imidazo [2,1-b] [1,3,5] benzothiadiazépine et ses sels.

6. La 5-[4-(2-hydroxyéthyl)-pipérazino]-imidazo [2,1-b] [1,3,5] benzothiadiazépine et ses sels.

7. Compositions pharmaceutiques contenant des composés selon la revendication 1 avec un véhicule pharmaceutique.

8. Les composés mentionnés dans la revendication 1, pour l'utilisation dans un procédé pour le traitement thérapeutique de l'organisme humain ou animal.

9. Les composés mentionnés dans la revendication 1, pour l'utilisation dans un produit servant lui-même dans un procédé pour le traitement thérapeutique de l'organisme humain ou animal.

10. Utilisation des composés mentionnés dans la revendication 1 pour la préparation de compositions pharmaceutiques.

11. Procédé de préparation des composés mentionnés dans la revendication 1, caractérisé en ce que :

a) on condense des composés de formule générale III ou leurs sels avec des composés de formule générale IV

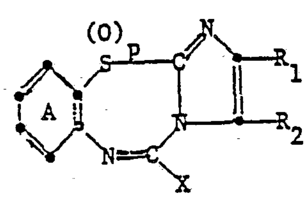

(III)

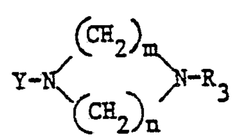

(IV)

X représentant un halogène, un groupe alcoxy inférieur, alkylthio inférieur, cyanato ou thiocyanato, Y l'hydrogène ou un atome de métal alcalin, p un nombre de 0 à 2, et les autres symboles ayant les significations indiquées en référence à la figure 1 dans la revendication 1, ou bien

b) on cyclise des composés de formule générale V

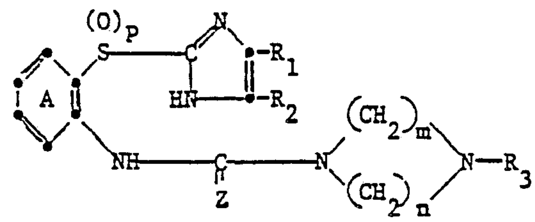

(V)

dans laquelle Z représente un atome d'oxygène ou de soufre ou un groupe NH et les autres symboles ont les significations indiquées ci-dessus, par déshydratation ou déshydrosulfuration, et lorsqu'on désire un composé de formule I dans laquelle $R_3$ représente un groupe alkyle en $C_1$-$C_7$, on introduit un groupe alkyle en $C_1$-$C_7$ dans un composé de formule I dans laquelle $R_3$ représente l'hydrogène ou un atome de métal alcalin, et/ou, lorsqu'on désire un composé de formule I dans laquelle $R_3$ représente l'hydrogène, on hydrolyse un composé obtenu dans lequel $R_3$ représente un reste acyle, et/ou, lorsqu'on désire un composé de formule I dans laquelle $R_3$ représente un groupe méthyle, et qu'on a obtenu un produit dans lequel $R_3$ représente un groupe (alcoxy en $C_1$-$C_7$)-carbonyle ou phényl-(alcoxy en $C_1$-$C_7$)-carbonyle, on réduit ce groupe en un groupe méthyle, et/ou, lorsqu'on désire un N- et/ou un S-oxyde, on oxyde un composé obtenu dans lequel $R_3$ n'est pas l'hydrogène, et/ou, lorsqu'on désire uniquement un N-oxyde, on soumet un composé obtenu, dans lequel $R_3$ n'est pas l'hydrogène, à oxydation spécifique, et/ou, lorsqu'on désire uniquement un S-oxyde, on soumet un composé obtenu dans lequel $R_3$ représente un reste acyle, à une S-oxydation et on élimine éventuellement le reste acyle, et/ou, si on le désire, on convertit un composé de formule I obtenu à l'état libre en un sel ou un sel obtenu en le composé libre de formule I ou en un autre sel, et/ou, si on le désire, on sépare un mélange d'isomères ou de racémates obtenus en les isomères où racémates individuels, et/ou si on le désire, on résout les racémates obtenus en les antipodes optiques.

12. Les composés qu'on peut obtenir par le procédé de la revendication 11.

**Revendications** (pour l'Etat contractant AT)

1. Procédé de préparation des 5-diazocycloalkyl-imidazo-[2,1-b] [1,3,5] benzothiadiazépines de formule générale I

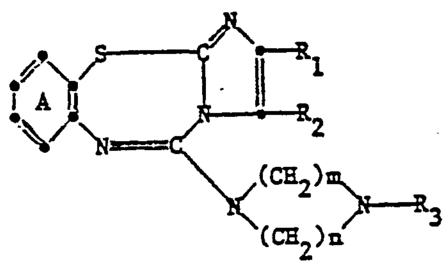

(I)

dans laquelle chacun des symboles $R_1$ et $R_2$ représente l'hydrogène, un groupe alkyle en $C_1$-$C_7$, alcanoyle en $C_1$-$C_7$, un halogène, un groupe cyano, carboxy, carbo-(alcoxy en $C_1$-$C_7$), carbamoyle, sulfamoyle, mono- ou di-(alkyle en $C_1$-$C_7$)-(carbamoyle ou sulfamoyle), le reste A est non substitué ou monosubstitué par un groupe alkyle en $C_1$-$C_7$, alcoxy en $C_1$-$C_7$, alkylthio en $C_1$-$C_7$, un halogène, un groupe trifluorométhyle, sulfamoyle, mono- ou di-(alkyle en $C_1$-$C_7$)-sulfamoyle, chacun des symboles m et n représente le nombre entier 2 ou 3 et $R_3$ représente l'hydrogène, un groupe alkyle en $C_1$-$C_7$, alcanoyle en $C_1$-$C_7$, (alcoxy en $C_1$-$C_7$)-carbonyle, phényl-(alcoxy en $C_1$-$C_7$)-carbonyle ou hydroxyalkyle en $C_1$-$C_7$, le groupe hydroxy étant séparé de l'atome d'azote par au moins deux atomes de carbone, et, lorsque $R_3$ représente un groupe alkyle en $C_1$-$C_7$ ou hydroxyalkyle en $C_1$-$C_7$, des N-oxydes correspondants en N-$R_3$ des composés de formule I, et/ou de leurs S-oxydes et des sels de ces composés, caractérisé en ce que :

a) on condense des composés de formule générale III ou leurs sels avec des composés de formule générale IV

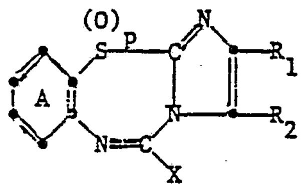 (III) 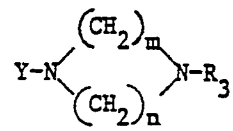 (IV)

X représentant un halogène, un groupe alcoxy inférieur, alkylthio inférieur, cyanato ou thiocyanato, Y l'hydrogène ou un atome de métal alcalin, p un nombre de 0 à 2, et les autres symboles ayant les significations indiquées en référence à la figure 1, ou bien

b) on cyclise des composés de formule générale V

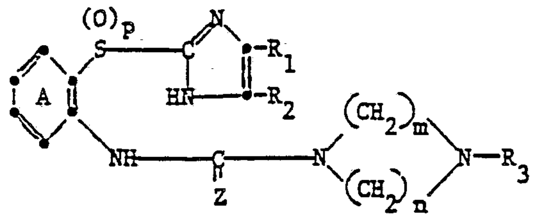 (V)

dans laquelle Z représente un atome d'oxygène ou de soufre ou un groupe NH et les autres symboles ont les significations indiquées ci-dessus, par déshydratation ou déshydrosulfuration, et lorsqu'on désire un composé de formule I dans laquelle $R_3$ représente un groupe alkyle en $C_1$-$C_7$, on introduit un groupe alkyle en $C_1$-$C_7$ dans un composé de formule I dans laquelle $R_3$ représente l'hydrogène ou un atome de métal alcalin, et/ou, lorsqu'on désire un composé de formule I dans laquelle $R_3$ représente l'hydrogène, on hydrolyse un composé obtenu dans lequel $R_3$ représente un reste acyle, et/ou, lorsqu'on désire un composé de formule I dans laquelle $R_3$ représente un groupe méthyle, et qu'on a obtenu un produit dans lequel $R_3$ représente un groupe (alcoxy en $C_1$-$C_7$)-carbonyle ou phényl-(alcoxy en $C_1$-$C_7$)-carbonyle, on réduit ce groupe en un groupe méthyle, et/ou, lorsqu'on désire un N- et/ou un S-oxyde, on oxyde un composé obtenu dans lequel $R_3$ n'est pas l'hydrogène, et/ou, lorsqu'on désire uniquement un N-oxyde, on soumet un composé obtenu, dans lequel $R_3$ n'est pas l'hydrogène, à oxydation spécifique, et/ou lorsqu'on désire uniquement un S-oxyde, on soumet un composé obtenu dans lequel $R_3$ représente un reste acyle, à une S-oxydation et on élimine éventuellement le reste acyle, et/ou, si on le désire, on convertit un composé de formule I obtenu à l'état libre en un sel ou un sel obtenu en le composé libre de formule I ou en un autre sel, et/ou, si on le désire, on sépare un mélange d'isomères ou de racémates obtenus en les isomères ou racémates individuels, et/ou si on le désire, on résout les racémates obtenus en les antipodes optiques.

2. Procédé selon la revendication 1, caractérisé en ce que l'on prépare des composés de formule II

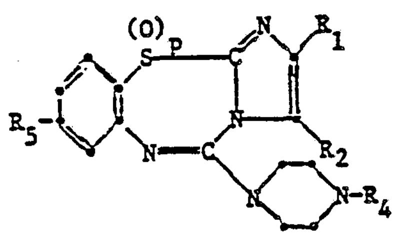 (II)

dans laquelle chacun des symboles $R_1$ et $R_2$ représente l'hydrogène ou un groupe alkyle en $C_1$-$C_7$, $R_4$ représente l'hydrogène, un groupe alkyle en $C_1$-$C_7$ ou 2- ou 3-hydroxyalkyle en $C_1$-$C_7$, $R_5$ représente l'hydrogène, un groupe alkyle en $C_1$-$C_7$, alcoxy en $C_1$-$C_7$, un halogène ou un groupe trifluorométhyle, et p est un nombre de 0 à 2, ainsi que les B-oxydes en $NR_4$ lorsque $R_4$ représente un groupe alkyle en $C_1$-$C_7$, et les sels de ces composés.

3. Procédé selon la revendication 1, caractérisé en ce que l'on prépare la 5-(4-méthylpipérazino)-imidazo [2,1-b] [1,3,5] benzothiadiazépine, la 8-chloro-5-(4-méthylpipérazino)-imidazo [2,1-b] [1,3,5] benzothiadiazépine, la 3-méthyl-5-(4-méthylpipérazino)-imidazo-[2,1-b] [1,3,5] benzothiadiazépine et la 5-[4-(2-hydroxyéthyl)-pipérazino]-imidazo [2,1-b] [1,3,5] benzothiadiazépine et leurs sels.

4. Procédé selon la revendication 1, pour la préparation des 5-diazacycloalkyl-imidazo [2,1-b] [1,3,5] benzothiadiazépines de formule I de la revendication 1, dans laquelle $R^3$ représente l'hydrogène, un groupe alkyle en $C_1$-$C_7$, alcanoyle en $C_1$-$C_7$, (alcoxy en $C_1$-$C_7$)-carbonyle ou hydroxyalkyle en $C_1$-$C_7$, dans lesquelles le groupe hydroxy est séparé de l'atome d'azote par au moins deux atomes de carbone, et les autres symboles ont les significations indiquées dans la revendication 1 ; de leurs N- et/ou S-oxydes selon la revendication 1, et de leurs sels, caractérisé en ce que :

a) on condense des composés de formule générale III ou leurs sels avec des composés de formule générale IV

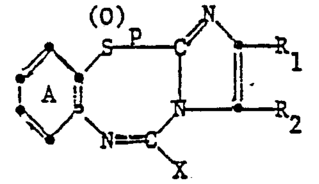 (III) 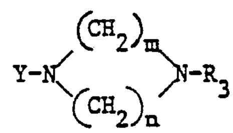 (IV)

dans lesquelles p est égal à 0, X représente un halogène, un groupe alcoxy inférieur, alkylthio inférieur, cyanato ou thiocyanato, Y représente l'hydrogène ou un atome de métal alcalin, chacun des symboles $R_1$ et $R_2$ représente l'hydrogène, un groupe alkyle en $C_1$-$C_7$, alcanoyle en $C_1$-$C_7$, un halogène, un groupe cyano, carbo-(alcoxy en $C_1$-$C_7$), carbamoyle, sulfamoyle, mono- ou di-(alkyle en $C_1$-$C_7$)-(carbamoyle ou sulfamoyle) et les autres symboles ont les significations indiquées en référence à la formule I ci-dessus, ou bien

b) on cyclise par déshydratation ou déshydrosulfuration des composés de formule générale V

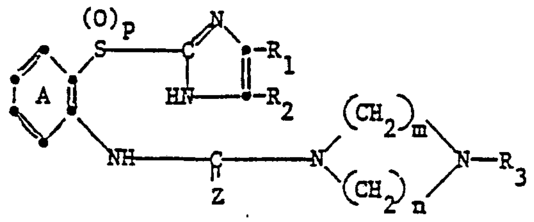 (V)

dans laquelle p est égal à 0, Z représente un atome d'oxygène ou de soufre ou le groupe NH et les autres symboles ont les significations indiquées ci-dessus et, lorsqu'on désire un composé de formule I dans laquelle $R_3$ représente un groupe alkyle en $C_1$-$C_7$ ou hydroxyalkyle en $C_1$-$C_7$, on introduit un tel reste dans un composé de formule I dans laquelle $R_3$ représente l'hydrogène ou un métal alcalin par réaction avec un ester réactif d'un alcanol inférieur ou d'un alcane-diol inférieur mono-estérifié, et/ou, si on désire un composé de formule I dans laquelle $R_3$ représente un reste acyle défini ci-dessus, on acyle un composé obtenu dans lequel $R_3$ représente l'hydrogène par réaction avec des dérivés réactifs des acides correspondants, et/ou, si on désire un N- et/ou un S-oxyde, on oxyde un composé obtenu dans lequel $R_3$ ne représente pas l'hydrogène, et/ou, si on désire uniquement un N-oxyde, on soumet un composé obtenu dans lequel $R_3$ ne représente pas l'hydrogène, à une oxydation spécifique, et/ou, si on le désire, on convertit un composé de formule I obtenu à l'état libre en un sel ou un sel obtenu en le composé libre de formule I ou en un autre sel, et/ou, si on le désire, on sépare un mélange d'isomères ou de racémates obtenu en les isomères ou racémates individuels, et/ou, si on le désire, on résout les racémates obtenus en les antipodes optiques.

5. Procédé selon la revendication 4, caractérisé en ce que l'on prépare des composés de formule I de la revendication 1, dans laquelle $R_3$ représente l'hydrogène, un groupe alkyle en $C_1$-$C_7$, alcanoyle en $C_1$-$C_7$, (alcoxy en $C_1$-$C_7$)-carbonyle ou hydroxyalkyle en $C_1$-$C_7$ et dans lesquels le groupe hydroxy est séparé de l'atome d'azote par au moins deux atomes de carbone, les autres symboles ayant les significations indiquées dans la revendication 2 ; leurs N- et/ou S-oxydes et leurs sels.

6. Procédé selon la revendication 4, caractérisé en ce que l'on prépare des composés de formule générale II

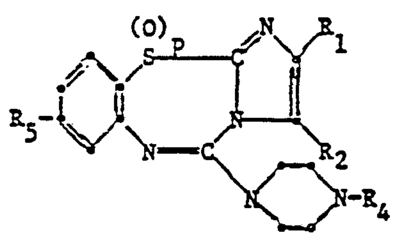 (II)

dans laquelle chacun des symboles $R_1$ et $R_2$ représente l'hydrogène, $R_4$ représente l'hydrogène, un groupe alkyle en $C_1$-$C_7$ ou 2- ou 3-hydroxyalkyle en $C_1$-$C_7$, $R_5$ représente l'hydrogène, un groupe alkyle en $C_1$-$C_7$, alcoxy en $C_1$-$C_7$, un halogène ou un groupe trifluorométhyle, et p est égal à 0, et les sels de ces composés.

7. Procédé selon la revendication 4, caractérisé en ce que l'on prépare la 5-(4-méthylpipérazino)-imidazo [2,1-b] [1,3,5] benzothiadiazépine, la 8-chloro-5-(4-méthylpipérazino)-imidazo [2,1-b] [1,3,5] benzothiadiazépine et la 5-[4-(2-hydroxyéthyl)-pipérazino]-imidazo [2,1-b] [1,3,5] benzothiadiazépine et leurs sels.

8. Procédé de préparation d'une composition pharmaceutique caractérisé en ce que l'on combine une substance active selon l'invention selon l'une des revendications 1 à 3 avec un véhicule pharmaceutique.

9. Procédé de préparation d'une composition pharmaceutique caractérisé en ce que l'on combine une substance active selon l'invention selon l'une des revendications 4 à 7 avec un véhicule pharmaceutique.